# EUROPEAN PATENT SPECIFICATION

(11) **EP 1 682 007 B1**
(45) Date of publication and mention of the grant of the patent: **03.10.2007**
(21) Application number: 04796314.5
(22) Date of filing: 25.10.2004
(51) Int. Cl.: A61B 17/00

(54) **PATENT FORAMEN OVALE CLOSURE SYSTEM**
VERSCHLUSSSYSTEM FÜR EIN OFFENES FORAMEN OVALE
SYSTEME DE FERMETURE DE FORAMEN OVALE

(30) Priority: 24.10.2003 US 514390 P
(43) Date of publication of application: 26.07.2006
(73) Proprietor: ev3 Endovascular, Inc., Plymouth, MN 55422 (US)
(72) Inventor: ADAMS, Daniel, O., Long Lake, Michigan 55356 (US); KUSLEIKA, Richard, S., Eden Prairie, Minnesota 55346 (US)
(74) Representative: Mallalieu, Catherine Louise
(86) International application number: PCT/US2004/035303
(87) International publication number: WO 2005/039419

(56) References cited:
- US-A1- 2001 039 436
- US-A1- 2002 183 787
- US-B1- 6 283 983

## Description

### Background of the Invention

### Field of the Invention

The present invention relates to devices for closing a body lumen or cavity and, in particular, for closing a patent foramen ovale.

### Description of the Related Art

Embolic stroke is the nation's third leading killer for adults, and is a major cause of disability. There are over 700,000 strokes per year in the United States alone. Of these, roughly 100,000 are hemorrhagic, and 600,000 are ischemic (either due to vessel narrowing or to embolism). About 50,000 of the ischemic strokes are believed to be caused by a patent foramen ovale. However, the risk of recurrent stroke is higher in patients whose strokes are caused by a patent foramen ovale.

Pharmacological therapies for stroke prevention such as oral or systemic administration of warfarin or the like have been inadequate due to serious side effects of the medications and lack of patient compliance in taking the medication.

In general, the heart is divided into four chambers, the two upper being the left and right atria and the two lower being the left and right ventricles. The atria are separated from each other by a muscular wall, the interatrial septum, and the ventricles by the interventricular septum.

Either congenitally or by acquisition, abnormal openings, holes or shunts can occur between the chambers of the heart or the great vessels (interatrial and interventricular septal defects or patent ductus arteriosus and aortico-pulmonary window respectively), causing shunting of blood through the opening. During fetal life, most of the circulating blood is shunted away from the lungs to the peripheral tissues through specialized vessels and foramens that are open ("patent"). In most people these specialized structures quickly close after birth, but sometimes they fail to close. A patent foramen ovale is a condition wherein an abnormal opening is present in the septal wall between the two atria of the heart. An atrial septal defect is a condition wherein a hole is present in the septal wall between the two atria of the heart.

In contrast to other septal defects which tend to have a generally longitudinal axis, a patent foramen ovale tends to behave like a flap valve. Accordingly, the axis of the patent foramen ovale tends to be at an angle, and almost parallel to the septal wall. The patent foramen ovale is a virtual tunnel, long and wide, but not very tall. It is normally closed because the roof and floor of the tunnel are in contact, but it can open when the pressure in the right side of the heart becomes elevated relative to the pressure in the left side of the heart, such as while coughing.

Studies have shown that adults with strokes of unknown origin (cryptogenic strokes) have about twice the normal rate of patent foramen ovales than the normal population. Although there is a correlation between strokes and patent foramen ovales, it is currently unknown why this correlation exists. Many people theorize that blood clots and plaque that have formed in the peripheral venous circulation (in the legs for example) break off and travel to the heart. Normally, the clots and plaque get delivered to the lungs where it is trapped and usually cause no harm to the patient. Patients with a patent foramen ovale, however, have a potential opening through which the clots or plaque can pass from the venous circulation and to the arterial circulation. The clots or plaque can then travel to the brain or other tissues to cause a thromboembolic event like a stroke. The clots may pass to the arterial side when there is an increase in the pressure in the right atrium. Then the clots travel through the left side of the heart, to the aorta, and then to the brain via the carotid arteries where they cause a stroke.

Previously, patent foramen ovale have required relatively extensive surgical techniques for correction. To date the most common method of closing intracardiac shunts, such as a patent foramen ovale, entails the relatively drastic technique of open-heart surgery, requiring opening the chest or sternum and diverting the blood from the heart with the use of a cardiopulmonary bypass. The heart is then opened, the defect is sewn shut by direct suturing with or without a patch of synthetic material (usually of Dacron, Teflon, silk, nylon or pericardium), and then the heart is closed. The patient is then taken off the cardiopulmonary bypass machine, and then the chest is closed.

In place of direct suturing, closure of a patent foramen ovale by means of a mechanical prosthesis has also been disclosed. A number of devices designed for closure of interauricular septal defects have been used to correct patent foramen ovale.

Although these devices have been known to effectively close other septal defects, there are few occlusion devices developed specifically for closing patent foramen ovale.

US2002/183787 discloses such a closure device to be delived through the septal defect by a delivery catheter, said closure device having a distal and proximal occlusion member for placement on each side of the defect, with an elongated member extending through the defect, joining the proximal and distal occlusion members.

### Summary of the Invention

Although the aforementioned devices have been effective in some cases, there is still much room for improvement, and there remains a need for a transluminal apparatus for correcting patent foramen ovale. Accordingly, disclosed herein are embodiments of various minimally invasive occlusion devices for closing a patent foramen ovale. Also disclosed is a delivery and positioning system.

A system for closing a patent foramen ovale is disclosed as comprising a delivery catheter with a proximal end and a distal end and a passageway extending therethrough. A tissue piercing structure may also be provided, having an opening and coupled to a first actuator. The tissue piercing structure may be slideable within the passageway of the delivery catheter and may be configured to pierce through tissue at the patent foramen ovale. The system may also comprise a closure device that is configured to be received within the opening of the tissue piercing structure. The closure device may comprise an elongate body that has proximal end and a distal end. The closure device may also comprise an expandable distal retaining portion at the distal end of the elongate body. The expandable distal retaining portion may have a portion thereof with a dimension that, when expanded, exceeds that of a portion of the tissue piercing structure and is configured to engage tissue on one side of the patent foramen ovale. The closure device may further comprise a proximal retaining portion that has an aperture that extends at least partially therethrough and is configured to engage the elongate body at its proximal end and is configured to engage tissue on another side of the patent foramen ovale. The system may further comprise a second actuator releasably engaged with the proximal end of the elongate body and adapted to move at least the elongate body and the distal retaining portion relative to the tissue piercing structure. Further, the proximal retaining portion may be configured to slide axially at least partially along the elongate body to engage the elongate body.

A device is also disclosed for closing a patent foramen ovale. The device may comprise an expandable distal retaining portion that is configured to be deliverable with a delivery catheter. At least a portion of the expandable distal retaining portion may have a width that, when expanded, exceeds that of a portion of the delivery catheter. The device may also comprise an elongate body having a distal and a proximal portion, and either the distal portion or the proximal portion of the elongate body may be configured to be coupled to the expandable distal retaining portion. The elongate body may comprise an elongate expandable portion that is configured to be in an unexpanded condition when placed within the delivery catheter. The expandable portion of the elongate member may be configured to expand when the distal and proximal portions of the elongate member are axially displaced toward each other.

For purposes of summarizing the invention, certain embodiments, advantages, and features of the invention have been described herein. It is to be understood that not necessarily all such embodiments, advantages, or features are required in any particular embodiment of the invention, and not all embodiments, advantages, or features are summarized above. Additionally, it is to be understood that this summary is not intended to limit in any way the embodiments, advantages, or features described below in the Detailed Description of the Preferred Embodiments or the Claims.

### Brief Description of the Drawings

FIG. 1 is an anterior illustration of a heart, with the proximal parts of the great vessels.

FIG. 2A is a side view of an occlusion device in accordance with one embodiment of the present invention.

FIGs. 2B-E are detailed views of the components of the occlusion device of FIG. 2A.

FIG. 3 is a schematic view of an occlusion device delivery catheter.

FIG. 4 is a schematic cross-sectional view of one embodiment of the distal end of the delivery catheter.

FIG. 5 is a detailed cross-sectional view of the proximal end of a delivery catheter.

FIGs. 6-11 are schematic views showing a method of delivery of the occlusion device of FIG. 2.

FIG. 12 is a schematic view of one embodiment of an anchor element coupled to an actuator.

FIG. 13 is a detailed cross-sectional view of one embodiment of the distal end of a delivery catheter.

FIG. 14 is a detailed cross-sectional view of another embodiment of the distal end of a delivery catheter.

FIG. 14A is a detailed cross-sectional view of another embodiment of the anchor element.

FIG. 15 is a schematic view of an occlusion device in accordance with another embodiment of the present invention.

FIG. 16 is a schematic view of an occlusion device in accordance with another embodiment of the present invention.

FIG. 17 is a schematic view of the occlusion device of FIG. 16 delivered at a treatment site.

FIG. 18 is a schematic view of an occlusion device delivered to a treatment site through the right atrium.

FIG. 19A is a schematic view of an occlusion device in accordance with another embodiment of the present invention in a compressed state.

FIG. 19B is a schematic view of the occlusion device of FIG. 19A in an expanded state.

FIG. 19C is a schematic view of an occlusion device in accordance with another embodiment of the present invention in a compressed state.

FIG. 19D is a schematic view of the occlusion device of FIG. 19C in an expanded state.

FIG. 19E is a schematic view of an occlusion device in accordance with another embodiment of the present invention in a compressed state.

FIG. 19F is a schematic view of the occlusion, device of FIG. 19E in an expanded state.

FIG. 19G is a schematic view of an occlusion device in accordance with another embodiment of the invention.

FIG. 19H is a schematic view of an occlusion device in accordance with another embodiment of the invention.

FIGS. 19I and 19J are cross-sections of the occlusion device of FIG. 19G.

FIGS. 19K and 19L are cross-sections of the occlusion device of FIG. 19H.

FIG. 20 is a perspective view of an embodiment of the tubular element of the device of FIG. 19A in an expanded state.

FIG. 21 is a schematic view of the occlusion device of FIG. 19A delivered at a treatment site.

FIG. 22A is a schematic view of an occlusion device in accordance with another embodiment of the present invention in a compressed state.

FIG. 22B is a schematic view of the occlusion device of FIG. 22A in an expanded state.

FIG. 23A is a schematic side view of a delivery device in accordance with another embodiment of the present invention.

FIG. 23B is a schematic end view of the delivery device of FIG. 23A.

FIG. 23C is a schematic end view of an alternative embodiment of the delivery device of FIG. 23A.

FIG. 24 is a schematic view of an occlusion device in accordance with another embodiment of the present invention.

FIGS. 24A and 24B are schematic views of the occlusion device of FIG. 24 attached to a push rod within a delivery catheter.

FIG. 25 is a schematic view of the occlusion device of FIG. 24 in an expanded state.

FIG. 26 is a schematic view of an occlusion device in accordance with another embodiment of the present invention.

FIG. 27 is a side view of the occlusion device of FIG. 26.

FIG. 28 is a schematic view of a delivery system for the occlusion device of FIG. 26.

FIGs. 29A and 29B are schematic views of the occlusion device of FIG. 26 delivered at a treatment site.

FIGs. 29C and 29D are schematic views of the occlusion device of FIG. 26 being delivered at a treatment site.

FIGs. 30 is a schematic view showing a method of delivery of the occlusion device of FIG. 24.

FIG. 31 is a schematic view of an occlusion device in accordance with another embodiment of the present invention.

FIG. 32 is a schematic view of an occlusion device in accordance with another embodiment of the present invention.

FIG. 33 is a schematic view of an occlusion device in accordance with another embodiment of the present invention.

FIGs. 34A and B are schematic views of the occlusion devices of FIGS. 32 and 33 delivered at a treatment site.

FIG 35A is a schematic view of an occlusion device in accordance with another embodiment of the present invention.

FIG. 35B is a cross-sectional schematic view of a delivery device for delivering the occlusion device of FIG. 35A

FIG. 35C is a cross-sectional view of the delivery device of FIG. 35B.

FIGs. 36A-B are schematic views of an occlusion device being delivered to a treatment site in accordance with an alternative embodiment.

FIG. 37A is a schematic view of an occlusion device in accordance with another embodiment of the present invention.

FIG. 37B is a cross-sectional view of the device of FIG. 37A.

FIG. 37C is a schematic view of a delivery device for delivering the device of FIG. 37A.

FIGs. 37D-F are schematic views of a method of delivering the device of FIG. 37A.

### Detailed Description of the Preferred Embodiments

For simplicity, the embodiments of the present invention will be described primarily in the context of a patent foramen ovale closure procedure. However, the device and methods herein are readily applicable to a wider variety of closure or attachment procedures, and all such applications are contemplated by the present inventors. For example, additional cardiac procedures such as atrial septal defect closure, ventricular septal defect closure, and atrial appendage closure are contemplated. Vascular procedures such as patent ductus arteriosis closure, isolation or repair of aneurysms, anastamosis of vessel to vessel or vessel to prosthetic tubular graft joints may also be accomplished using the devices as described herein. Attachment of implantable prostheses, such as attachment of the annulus of a prosthetic tissue, mechanical heart valve or an annuloplasty ring may be accomplished. A variety of other tissue openings, lumens, hollow organs and surgically created passageways may be closed in accordance with the preferred embodiments. Closures and repairs described herein may be accomplished using catheter based interventional methods or minimally invasive surgical methods. Adaptation of the devices and methods disclosed herein to accomplish procedures such as the foregoing will be apparent to those of skill in the art in view of the disclosure herein.

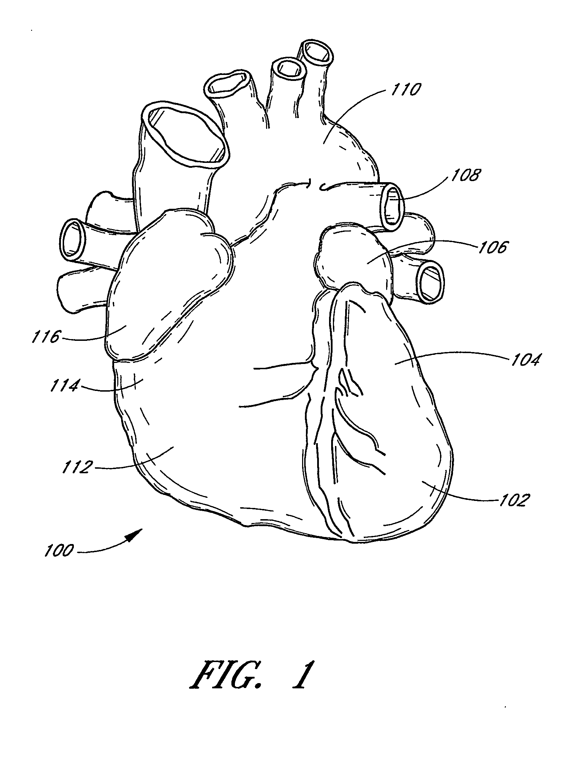

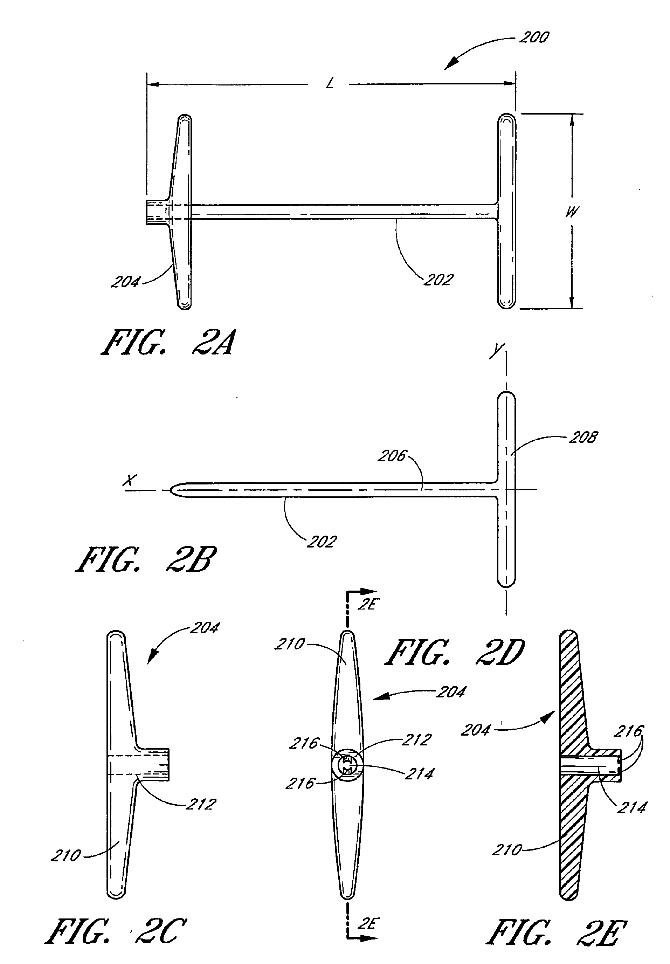

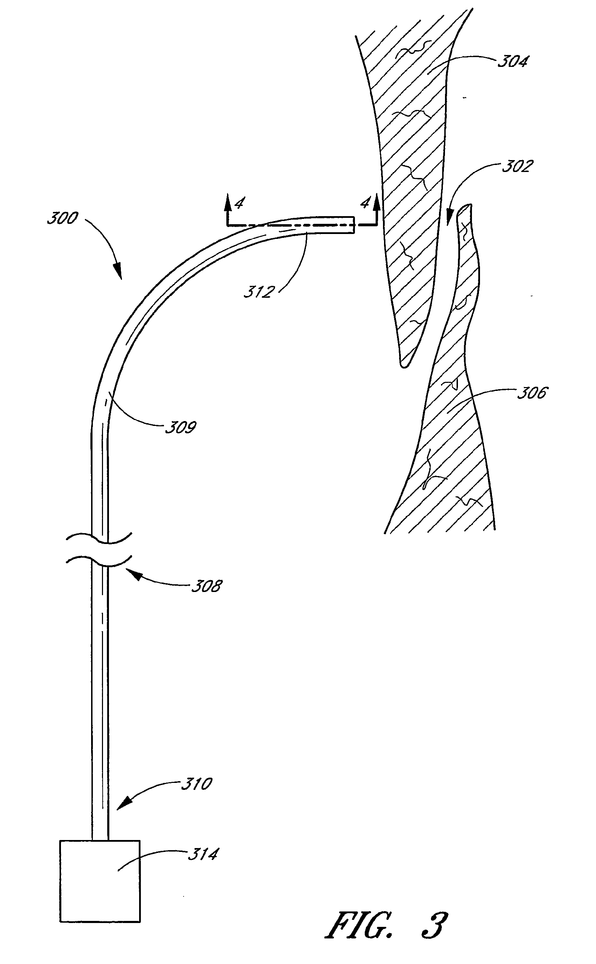

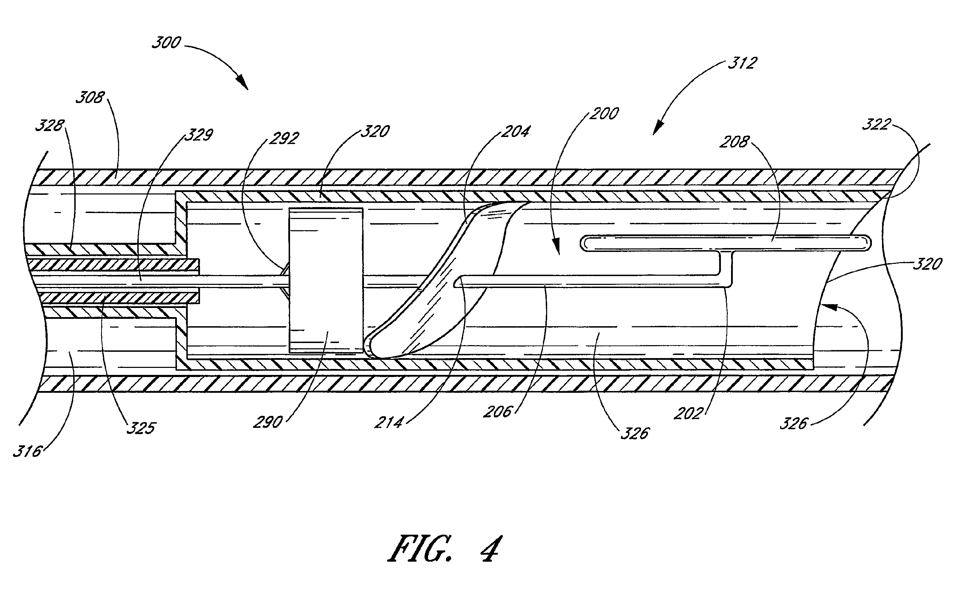

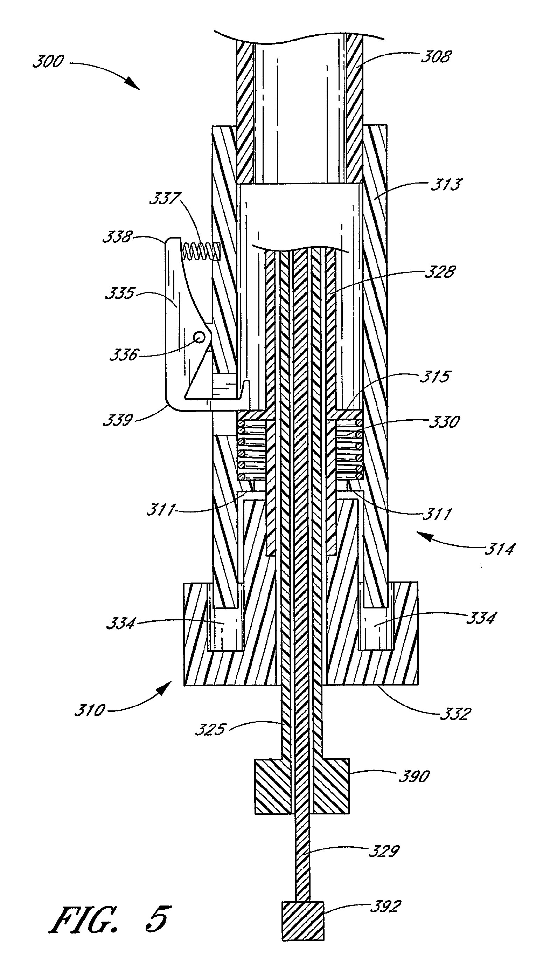

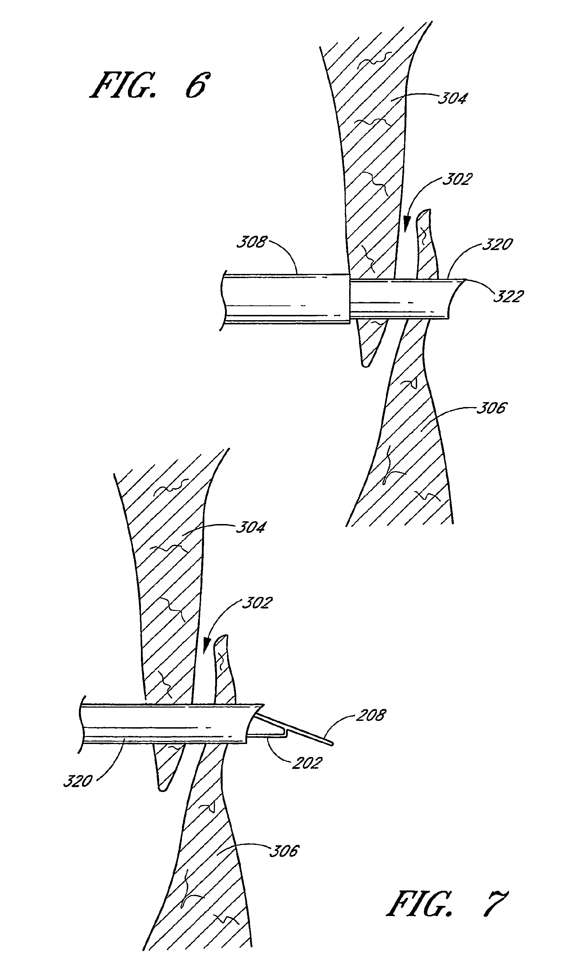

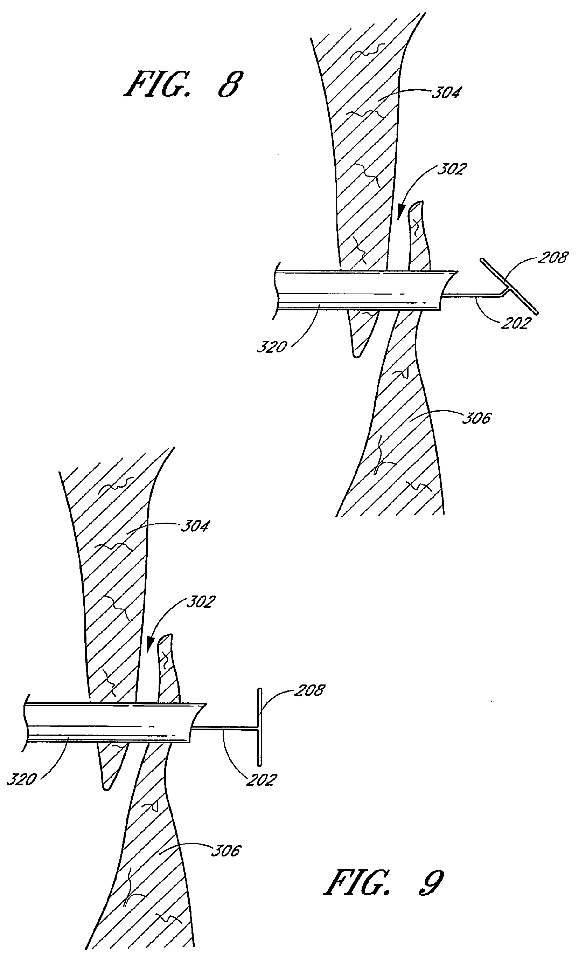

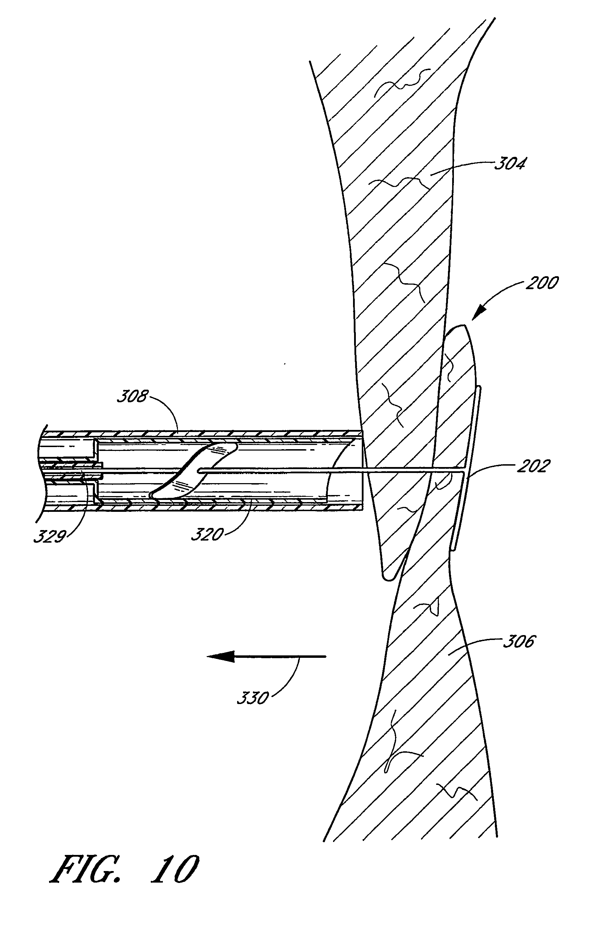

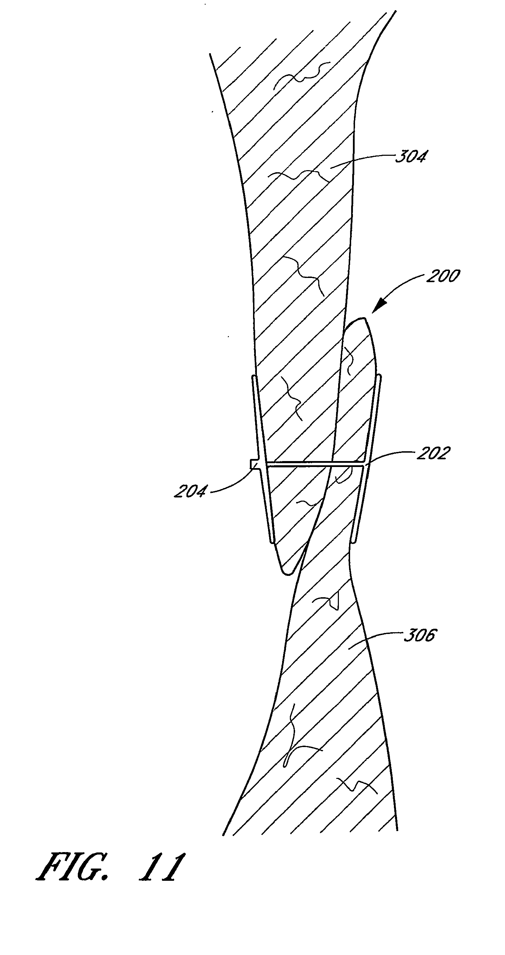

Referring to FIG. 1, a heart 100 is illustrated to show certain portions including the left ventricle 102, the left atrium 104, the left atrial appendage 106, the pulmonary artery 108, the aorta 110, the right ventricle 112, the right atrium 114, and the right atrial appendage 116. As is understood in the art, the left atrium 104 is located above the left ventricle 102 and the two are separated by the mitral valve (not illustrated).

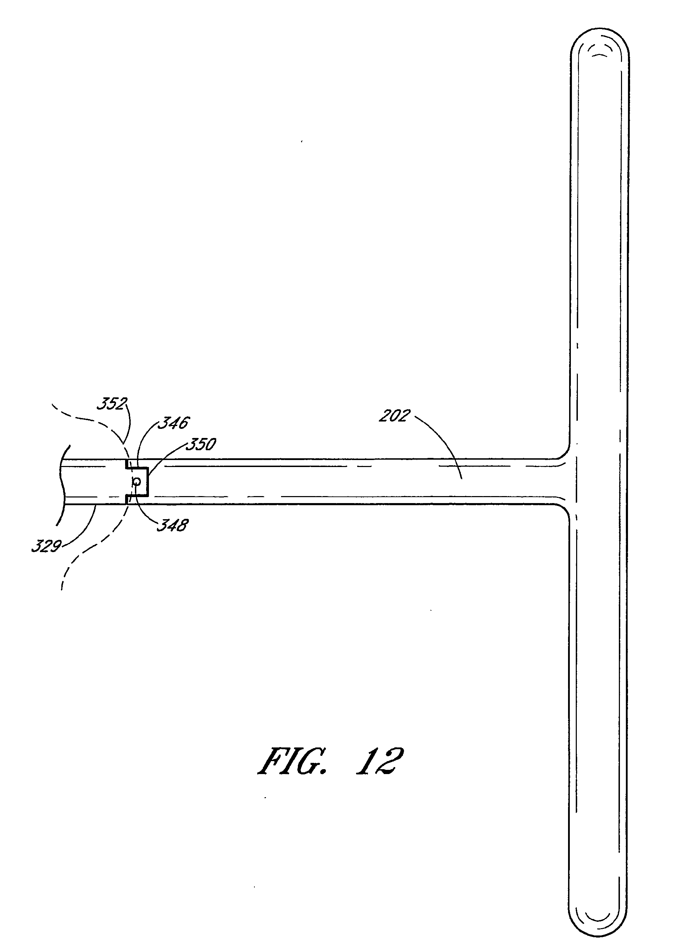

Referring to FIG. 2A, there is illustrated one embodiment of an occlusion device 200 in accordance with the present invention. It will be appreciated that the term "occlusion" should not be limited to mean full occlusion, as partial occlusion can also satisfactorily accomplish occlusion. The term "occlusion" as used herein is a broad term and is intended to encompass any structure capable of providing an effective barrier within a patent foramen ovale or other opening to be closed or at least partially blocked. The occlusion device 200 includes an anchor element 202 and a retention element 204. The occlusion device 200 is shown as being generally "I"-shaped. Although the device is shown having an "I"-shape, it is envisioned that a number of variations of this shape can be utilized to provide the same results.

In one embodiment, the occlusion device 200 may be made from a medical plastic or a metal, such as stainless steel, Nitinol, Elgiloy®, polyester, PEEK^{™} or others which can be determined through routine experimentation by those of skill in the art. In another embodiment, the occlusion member 200 may be made of a dissolvable suture material. The occlusion device 200 may also be biodegradable. It is also envisioned that other metallic or non-metallic biocompatible materials may be used to form occlusion device 200.

The occlusion device 200 may have a circular, rectangular, or other shaped cross-section, depending upon the manufacturing technique. In one embodiment, a circular cross section is molded from a biocompatible polymer, such as polyethylene terephthalate (PET).

For use in a patent foramen ovale, in one embodiment, the overall width W of the occlusion device 200 may be any value or range of values from about 1 cm to about 5 cm, and, in one more preferred embodiment, is about 1.0 cm. In some embodiments, the overall width of the device 200 may be significantly less than about 1 cm or significantly greater than about 5 cm. The overall length L of the occlusion device 200 from the distal end to the proximal end in one embodiment may be any value or range of values from about 4 mm to about 20 mm and, in one more preferred embodiment, is about 12 mm. In some embodiments, the overall length L of the device 200 may be significantly less than about 4 mm or significantly greater than about 20 mm.

As shown in Figure 2B, the anchor element 202 has an elongate body portion 206 and a retaining portion 208. In one embodiment, the longitudinal axis x of the elongate body portion 206 is perpendicular to the longitudinal axis y of the retaining portion 208. In some embodiments, the angle between the longitudinal axis x of the elongate body portion 206 and the longitudinal axis y of the retaining portion 208 may vary. In some embodiments, the angle between the axis x and the axis y may be any value or range of values between about 15 and 165 degrees. In other embodiments, the angle value or range of values can vary below about 15 degrees and above about 165 degrees. The anchor element 202 may have a circular, rectangular, or other shaped cross-section, depending upon the manufacturing technique.

Referring to Figures 2C-2E, the retention element 204 includes a retaining portion 210 and a receiving portion 212 which, in some embodiments, extends outwardly from the retaining portion 210. The receiving portion 212 may include a lumen or opening 214 for receiving the anchor element 202. The opening 214 may also include imbedded tines 216 which allow motion distally over the elongate body portion 206 of the anchor element 202, but grab and hold the elongate body portion 206 if proximal motion is attempted. Alternatively or additionally, the receiving portion 212 may include a plurality of teeth (not shown) within the lumen or opening 214 to prevent movement. In some embodiments, the anchor element 202 may have notches or indentations (not shown) to facilitate engagement of the tines 216, teeth, or other engaging devices on the retaining portion 210.

The occlusion device 200 is designed in one embodiment to be implanted using a delivery catheter. The device is designed to remain in a collapsed state while in the catheter, as described hereinbelow. Upon delivery to the patent foramen ovale, the device 200 is positioned and expanded to occlude the patent foramen ovale, as described hereinbelow. The device is preferably loaded into or onto the catheter prior to deployment, and is then deployed when properly positioned. The procedure for placing the occlusion device and delivery device will be described in further detail hereinafter.

In accordance with one embodiment of the present invention, an occlusion device delivery system may be provided for delivery of an occlusion device to a patent foramen ovale or other septal defect.

Referring to Figure 3, a delivery device 300 delivers the occlusion device to the patent foramen ovale 302. The patent foramen ovale 302 generally includes a septum secundum 304 and a septum primum 306. The delivery device 300 comprises a catheter 308 having an elongate flexible tubular body 309 extending between a proximal end 310 and a distal end 312. The catheter is shown in a highly schematic form, for the purpose of illustrating the functional aspects thereof. The catheter body will have a sufficient length and diameter to permit percutaneous entry into the vascular system, and transluminal advancement through the vascular system to the desired deployment site. For example, in an embodiment intended for access at the femoral artery and deployment within the left atrium, the catheter 308 will have a length within the range of from about 50 cm to about 150 cm, and a diameter of generally about or less than about 15 French. In some embodiments, the length of the catheter 308 may be less than about 50 cm or greater than about 150 cm, and the diameter of the catheter may be more than about 15 French. Further dimensions and physical characteristics of catheters for navigation to particular sites within the body are well understood in the art and will not be further described herein.

The flexible body can be manufactured in accordance with any of a variety of known techniques. In one embodiment, the flexible body 309 is extruded from any of a variety of materials such as HDPE, PEBAX®, nylon, and PEEK^{™}. Alternatively, at least a portion of or all of the length of the tubular body may comprise a spring coil, solid walled hypodermic needle or other metal tubing, or a braided reinforced wall, as are known in the art. The spring coil, tubing, braided reinforcement, or other structures may be encapsulated with thermoset polymers such as polyimide or with thermoplastic polymers such as PEBAX®, and the like.

The tubular body 309 may be provided with a handle 314 generally on the proximal end 310 of the catheter 308. The handle 314 may be provided with a plurality of access ports. The handle 314 may be provided with an access port which may be used as a guidewire port in an over the wire embodiment, and a deployment wire port. Additional access ports, such as a contrast media introduction port, or others may be provided as needed, depending upon the functional requirements of the catheter. The catheter 308 may be constructed to contain the same number of ports as the handle 314. The handle 314 permits manipulation of the various aspects of the occlusion device delivery system 300, as will be discussed below. Handle 314 may be manufactured in any of a variety of ways, typically by injection molding, machining or otherwise forming a handpiece for single-hand operation, using materials and construction techniques well known in the medical device arts.

Figure 4 is a schematic cross-sectional view of one embodiment of the distal end 312 of the delivery device 300. The catheter 308 may include a passageway 316 for delivery of the occlusion device 200 to the patent foramen ovale. The distal end 312 of the delivery device 300 may include a tissue piercing structure 320, such as a needle, provided therein. The tissue piercing structure 320 may have a tubular body, such that an opening 326 is formed in which the anchor element 202 and retention element 204 are provided. The tissue piercing structure 320 may have a pointed end 322 for accessing the patent foramen ovale, as will be described below. The tissue piercing structure 320 may be attached to or integral with a first actuator 328, as will be described below.

A second actuator 329, coupled to the anchor element 202, and optionally a third actuator 325, coupled to or engagable with the retention element 204 or optional lock 290, may be used to deliver and deploy the occlusion device 200 at a treatment site, as described further below. When in the delivery configuration the retention element 204 and lock 290 may be provided over the actuator 329 (see Figure 10) and be slideable relative thereto. Alternatively, the retention element 204 and lock 290 may in the delivery configuration be provided over the elongate body 206 (see Figure 4) and be slideable relative thereto. Any of a variety of structures such as polymeric or metal single or multiple strand wires, ribbons, or tubes can be used for the actuators 328, 329, 325. The actuators 328, 329, 325 may be retracted as with a pull wire design and/or rotated as with a torque rod design, and the like, as will be described herein.

In one embodiment, the anchor element 202 is bent so that the retaining portion 208 is compressed and substantially parallel to the elongate body portion 206 or longitudinal axis x, and then placed within the tissue piercing structure 320, which may have an end opening as shown, or a side opening (not shown). The anchor element 202, in other embodiments, may be at an acute angle with respect to the longitudinal axis x. The catheter 308 also desirably includes a central lumen 316 through which the anchor element 202 and retention element 204 are delivered to the septum, preferably when within the tissue piercing structure 320 which is provided in the central lumen 316. The anchor element may be locked or wedged or otherwise attached near the base of the tissue piercing structure. In a preferred embodiment, the anchor element 202 comprises a release as described in connection with Figure 12 below. In one embodiment, the anchor element 202 may be side-loaded.

Figure 4 also shows the retention element 204 having an aperture or opening 216 through which the elongate body 206 of the anchor element 202 passes through. The retention element 204 is bent to a compressed state when placed within the tissue piercing structure 320. The retention element 204 is configured to self expand when deployed at a treatment site. Although the retention element 204 is shown as elliptically-shaped, it is contemplated that the retention element 204 may be circular, rectangular, pentagonal, or other shapes. In some embodiments, the retention element 204 may be adjustable, such that the distance between the retention element 204 and the retaining portion 208 of the occlusion element 202 can vary. In some embodiments, a locking element 290 may be provided to lock the retention element 204 in place. The locking element 290 may be provided with tines 292 for locking the retention element 204 and anchor element 202 at a desired location.

The tissue piercing structure 320 may be spring loaded, but may also be advanced through the septum manually. In one embodiment, shown in Fig. 4, the proximal end of the tissue piercing structure 320 may be connected to actuator 328 contained within lumen 316. The actuators 328, 329, 325 may be stainless steel or nitinol or a polymer, such as high-density polyethylene (HDPE) or metal braid reinforced polyimide. The actuator 328 may be welded or bonded or crimped or otherwise affixed to the tissue piercing structure 320.

Actuators 328, 329, 325 may be a solid wire or a hypotube. In a preferred construction, shown in Fig. 4, actuator 328 may be integrally attached to the distal end or other distal point of attachment of the tissue piercing structure 320. Actuator 329 may be removably attached to the proximal end of the anchor element 202, and actuator 325 may or may not be attached to lock 290 or retention element 204. First actuator 328 may be attached to the tissue piercing structure by any technique such as welding, brazing, interference fit such as threaded fit or snap fit, adhesives, crimping, and the like. The actuators 328, 329 may comprise a variety of structures which have sufficient lateral flexibility to permit navigation of the vascular system, and sufficient axial column strength to manipulate the tissue piercing structure 320, and occlusion element 202, respectively. Any of a variety of structures such as hypotube, solid core wire, "bottomed out" coil spring structures, or combinations thereof may be used, depending upon the desired performance of the finished device. In one embodiment, the actuators 328, 329, 325 comprise stainless steel tubing. In some embodiments, actuator 325 may comprise a solid wire.

As used herein, the term "actuator" is a broad term and is intended to include any of a wide variety of structures that are capable of transmitting axial tension or compression such as a pushing or pulling force with or without rotation from the proximal end 310 to the distal end 312 of the catheter 308. Thus, monofilament or multifilament metal or polymeric rods or wires, woven or braided structures may be utilized. The actuator may also be reinforced with polymers. The actuator may also be formed with composite materials. Alternatively, tubular elements such as a concentric tube positioned within the outer tubular body 309 may be used, as will be apparent to those of skill in the art.

In the illustrated embodiment, the first actuator 328 is integral with or connected to the proximal end of the tissue piercing structure 320, while the second actuator 329 is releasably connected to the proximal end of the anchor element 202, and actuator 325 is provided near the proximal end of the retention element 204 (or optional lock 290). This permits axial movement of the anchor element 202 and retention element 204 relative to the tissue piercing structure 320. In particular, second actuator 329 is used to advance the anchor element 202 relative to the tissue piercing structure 320, and third actuator 325 is used to advance the retention element 204 relative to the tissue piercing structure 320. In some embodiments, the second actuator 329 may be used to advance the optional lock 290 as well. In other embodiments, the second actuator 329 may be used to advance only the optional lock 290.

Figure 5 shows a detailed cross-sectional view of one embodiment of the proximal end 310 of the delivery device 300 which will be provided outside of the patient, with actuators 328, 329, and 325 illustrated. In some embodiments, actuator 328 comprises an actuator flange 315. The delivery device 300 may include a trip lever 335 that holds a spring 330 in a compressed state. The spring 330 is long at rest, and axially compressing the spring stores energy. In some embodiments, the distal end of spring 330 is attached to the proximal end of actuator 328. In some embodiments, the distal end of spring 330 abuts the proximal end of actuator flange 315. The trip lever 335 may be attached to the proximal end 310 of the delivery device 300 at pivot 336. The pivot 336 may be attached to the handle body 313. The handle body may be made of a metal or polymer, such as stainless steel, nylon, Delrin, and the like. The handle body may be manufactured in any of a variety of ways, such as machining, molding, and the like.

The trip lever 335 has a distal end 338 and a proximal end 339. The proximal end 339 of the lever 335 may extend inside the handle body 313. The proximal end 339 of the lever 335 contacts the actuator flange 315. Pushing on the distal end 338 of the trip lever 335 results in a clockwise rotation (when viewed from the perspective of Figure 5) of the proximal end 339, thereby disengaging the trip lever 335 from the actuator flange 315, allowing the spring to advance actuator 328 and tissue piercing structure 320 distally. Nubs 311 may be provided on the interior surface of the catheter 308 to limit the motion of the spring 330 in a proximal direction, allowing the spring to be axially compressed.

A handle 332 having openings 334 is attached to actuator 328. The handle 332 is provided to retract the actuator 328 and tissue piercing structure 320. Proximally drawing handle 332 relative to handle body 313 proximally draws actuator 328, thereby compressing the spring 330 and allowing the proximal end 339 to return to the resting position via the trip lever spring 337 and pivot 336, wherein the spring 330 is compressed. Openings 334 in handle 332 may limit the advancement of actuator 328 and tissue piercing structure 320, by contacting the proximal end of handle body 313 with handle 332. The depth of openings 334 may be adjustable to vary the distance the tissue piercing structure 320 may be advanced. In an adjustable embodiment, the handle 332 may comprise a proximal component, and distal component (not shown), which have a threaded connection (not shown) to vary the depth of the openings 334.

Actuator 329 extends through handle 310 within actuator 328 and within optional actuator 325 and terminates in an optional handle 392. Optional actuator 325 extends through handle 310 within actuator 328 and terminates in an optional handle 390. The proximal end of actuator 329 and/or actuator 325 may be connected to any of a variety of actuator controls including rotational knobs, levers and sliders switches, and the like. In some embodiments, the actuator controls may be attached to handle body 313.

In some embodiments, the exterior surface of handle body 313 may be provided with a system for indicating the axial position of the tissue penetrating structure. In one embodiment, the system comprises a color coded system. In such an embodiment, the exterior surface may be provided with a red colored section and a green colored section. In one embodiment, the green colored section may be visible when the tissue piercing structure is entirely within the catheter 308, while the red colored section may be visible when the tissue piercing structure at least partially extends outside the catheter 308.

A method of delivering the occlusion device 200 to a treatment site is shown in Figures 6-11. In use, the deployment catheter 308 is percutaneously introduced into the vascular system and transluminally advanced into the heart and, subsequently, to the patent foramen ovale using techniques which are known in the art.

In accordance with some embodiments of the present invention, the delivery catheter 308 with modifications apparent to those of skill in the art in view of the intended application, may be utilized to close any of a variety of tissue apertures using the occlusion devices as described herein. These include, for example, atrial septal defects, ventricular septal defects, patent ductus arteriosis, and others which will be apparent to those of skill in the art.

The patent foramen ovale may be accessed via catheter through a variety of pathways. In one embodiment, the patent foramen ovale may be accessed from the venous circuit. The catheter may be introduced into the venous system, advanced into the inferior vena cava or superior vena cava and guided into the right atrium. The catheter may then be directed to the patent foramen ovale. Alternatively, once in the right atrium, the catheter may be advanced through the tricuspid valve and into the right ventricle and directed to a ventricular septal defect and the occlusion device deployed.

Alternatively, the patent foramen ovale may be accessed from the arterial circuit. The catheter is introduced into the arterial vascular system and guided up the descending thoracic and/or abdominal aorta. The catheter may then be advanced into the left ventricle through the aortic outflow tract. Once in the left ventricle, the catheter may be directed up through the mitral valve and into the left atrium. When the catheter is in the left atrium, it may be directed into the patent foramen ovale and the occlusion device deployed.

The occlusion device is preferably positioned within a septal defect to be occluded, such as a patent foramen ovale. Initially, the device is collapsed inside a delivery catheter 308, as shown in Figures 3-5, preferably within a tissue piercing structure 320 within the distal end of the catheter 308. The delivery system 300 is positioned at or near the patent foramen ovale, as shown in Figure 3.

The delivery device 300 is delivered to the patent foramen ovale 302 and the tissue piercing structure 320 is advanced distally through the septum secundum 304 and septum primum 306 by actuating first actuator 328, as shown in Figure 6. In some embodiments, tissue piercing structure 320 may be advanced across the septa manually. In other embodiments, tissue piercing structure 320 may be advanced across the septa using a spring loaded handle, as shown in Figure 5. Crossing the septa quickly using a spring loaded handle may facilitate crossing of the septum primum 306. Septum primum 306 may be thin and may tent when the tissue piercing structure 320 contacts the septum primum 306 through manual advancement. This problem occurs especially with aneurismal septa primum.

Referring to Figure 7, the anchor element 202 is advanced distally from the tissue piercing structure 320 by actuating second actuator 329.

Once the anchor element 202 exits the tissue piercing structure 320, the anchor element 202 self-expands into its expanded state from the compressed state, as shown in Figures 8 and 9. The occlusion device deployment system 300 permits the anchor element 202 to be maintained in a low crossing profile configuration, to enable transluminal navigation to a deployment site. Alternatively, certain embodiments of the anchor element 202 can be enlarged under positive force, such as by a mechanical mechanism.

In one embodiment, prior to deployment, the retaining portion 208 is configured inside the delivery system 300 parallel with or at an acute angle with respect to the anchor element 202. As the retaining portion 208 exits the delivery system 300, the retaining portion 208 is permitted to assume an unconstrained orientation that is substantially perpendicular to axis x or at an acute angle with respect to axis y. In this embodiment, the retaining portion 208 may assume an unconstrained orientation that increases its width W and may assume a constrained orientation that decreases its width W for deployment by the delivery system 300.

With reference to Figure 10, the tissue piercing structure 320 may be retracted and the anchor element 202 manipulated to draw the septum secundum 304 and septum primum 306 toward one another. By withdrawing the tissue piercing structure 320 proximal to the septum secundum 304 and by pulling proximally on actuator 329 attached to the anchor element 202 while pushing distally on the catheter shaft 308 and/or handle body 313 against the septum secundum 304, the anchor element 202 moves proximally to compress the septum secundum 304 and septum primum 306, thereby closing the patent foramen ovale and stopping unintended blood flow. The retention element 204 is secured in position with the anchor element 202 of the occlusion device 200 by distally advancing actuator 325. This moves the retention element 204 distally over the elongate body 206 until the retention element 204 engages the septum secundum 304. Depending on the starting location of the retention element 204 (Figure 4), in some embodiments, the retention element 204 may slide first over actuator 329 before engaging the elongate body 206. As discussed above, the retention element 204 may have teeth or other structure that permit distal movement but prevent proximal movement. Alternatively, in some embodiments, optional lock 290 (Figure 4) as discussed above may be used in combination with the retention element 204 to secure the occlusion device 200 in position. In some embodiments, wherein actuator 325 is omitted, actuator 328 may be attached to the tissue piercing structure 320, and advanced distally to secure the retention element 204 and optional lock 290 in position.

The retention element 204 may be delivered to seal the foramen ovale by sliding distally along the elongate body 206 to pinch or compress the septum sec undum 304 and septum primum 306 together, in combination with the anchor element 202. In one embodiment, retention element 204 is also desirably delivered in a compressed state and self-expands into an expanded state upon placement at the patent foramen ovale. Actuator 329 carrying the anchor element 202 may be tensioned while actuator 325 and retention element 204 may be delivered to the treatment site. In some embodiments, anchor element 202 also carries a lock 290, as described above.

After optimal positioning and sealing is achieved, anchor element 202 can be released from the actuator 329 and the delivery device 300 can be removed, as shown in Figure 11. Any excess length of the anchor element 202 proximal to the retention element 204 may be removed, such as by cutting.

Figure 12 shows one embodiment of the distal connection of the actuator 329 to the anchor element 202. The proximal end of the anchor element 2 02 may be provided with an opening 350 that is configured to receive a corresponding attachment element 346. The anchor element 202 and attachment element 346 include an opening 348 through which a small diameter wire or string member 352 can pass through to connect the anchor element 202 and the attachment element 346. If the attachment element 346 is located distal of the retention element 204 before deployment, the string member 352 may pass through the lumen or opening 214 of the retention element 204. The string member 352 axially extends through the catheter body 308 to a control means at the proximal end of the catheter body. By manipulating the string member 352, the string member 352 can be withdrawn from the opening 348, thereby releasing anchor element 202.

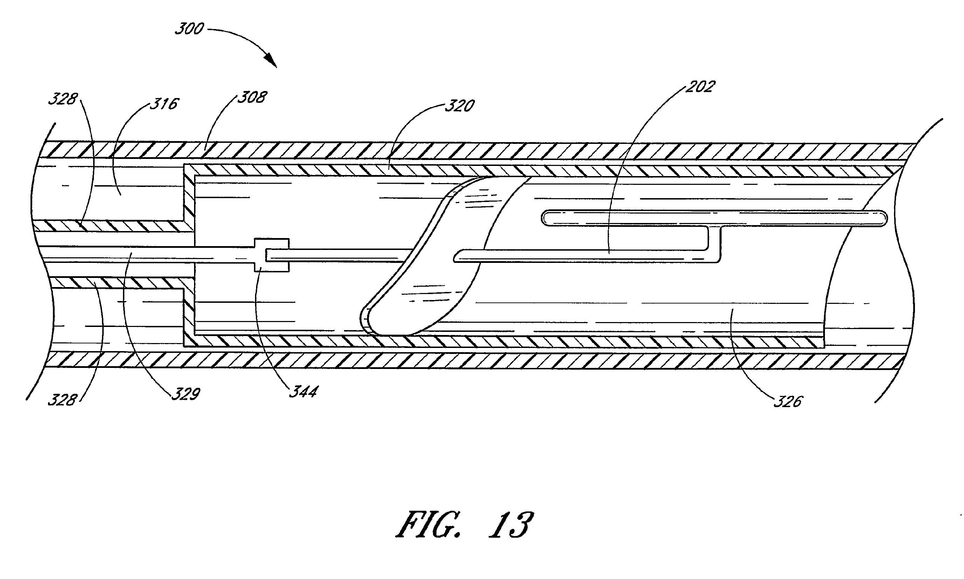

Figure 13 shows one embodiment of the distal end of the delivery device 300, illustrating an alternate method of releasing anchor element 202 from actuator 329. The second actuator 329 is shown attached to the anchor element 202 with attachment element 344. The attachment element 344 may include a threaded aperture through which the occlusion element 202 is threadably engaged. Any means known may be used for attaching the occlusion element 202 to actuator 329. For example, any of a variety of bonding techniques for dissimilar materials may be utilized, such as adhesives and various molding techniques. In one embodiment, heat is applied to the attachment element 344 to detach anchor element 202.

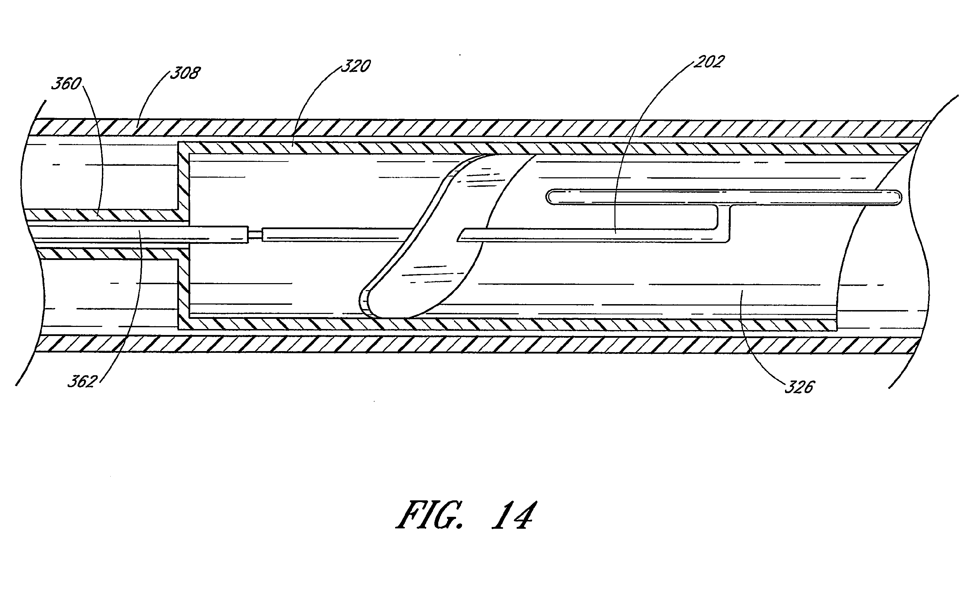

In an alternate construction, the anchor element 202 may be delivered and manipulated by rotating a torque element extending throughout the deployment catheter 308. Referring to Figure 14, the elongate flexible tubular body 308 includes an actuator 360 extending axially therethrough. A rotatable torque rod 362 extends axially through the actuator 360. The actuator 360 may be integrally formed with or otherwise attached to the tissue piercing element 320. The rotatable torque rod 362 is releasably attached to the anchor element 202, such as with screw threading.

The proximal end of the torque rod 362 may be connected at a proximal handle to a manual rotation device, such as a hand crank, thumb wheel, rotatable knob or the like. Alternatively, the torque rod may be connected to a power driven source of rotational energy such as a motor drive or air turbine.

The distal end of the torque rod may be integral with or connected to a rotatable core, which may extend axially into the elongate body portion 206 of the anchor element 202. The terms "torque rod" or "torque element" as used herein are broad terms and are intended to include any of a wide variety of structures which are capable of transmitting a rotational torque throughout the length of a catheter body. For example, solid core elements such as stainless steel, nitinol or other nickel titanium alloys, or polymeric materials may be utilized. The actuator 360 may be provided with an axially extending central lumen for receiving the torque rod 362. In an embodiment intended for implantation over a guide wire, the torque rod 362 may be provided with an axially extending central guidewire lumen. This may be accomplished by constructing the torque rod from a section of hypodermic needle tubing, having an inner diameter greater than the outside diameter of the intended guidewire. Tubular torque rods may also be fabricated or constructed utilizing any of a wide variety of polymeric constructions which include woven or braided reinforcing layers in the wall. Torque transmitting tubes and their methods of construction are well understood in the intracranial access and rotational atherectomy catheter arts, among others, and are not described in greater detail herein.

Upon placement of the anchor element 202 at the desired implantation site, the torque rod is rotated in a direction that produces an axial proximal retraction of torque rod 362 relative to anchor 202. Continued rotation of the torque rod will cause the threaded core to exit proximally through a threaded aperture which may be provided in the anchor element 202. At that point, the deployment catheter 308 may be proximally retracted from the patient, leaving the occlusion element 202 in place.

The actuator 360 may be provided with an antirotation lock (not shown) between a distal end of the actuator 360 and the proximal end of the occlusion element 202. In general, the anti-rotational lock may be conveniently provided by cooperation between a first surface on the distal end of the actuator 360, which engages a second surface on the proximal end of the anchor element 202, to rotationally link the actuator 360 and the anchor element 202. Any of a variety of complementary surface structures may be provided, such as an axial extension on one of the first and second surfaces. Such extensions and recesses may be positioned laterally offset from the axis of the catheter. Alternatively, they may be provided on the longitudinal axis with any of a variety of axially releasable anti-rotational couplings having at least one flat such as a hexagonal or other multifaceted cross sectional configuration.

The proximal end of the anchor element 202 may be provided with a threaded aperture through which the torque rod is threadably engaged. Alternatively, the torque rod may be provided with a threadable aperture through which the occlusion element 202 is threadably engaged.

With reference to Figure 14A, the anchor element 202 may be provided with a guide wire lumen 396. In an embodiment as shown in Figure 14A, the occlusion device may be delivered over a guide wire.

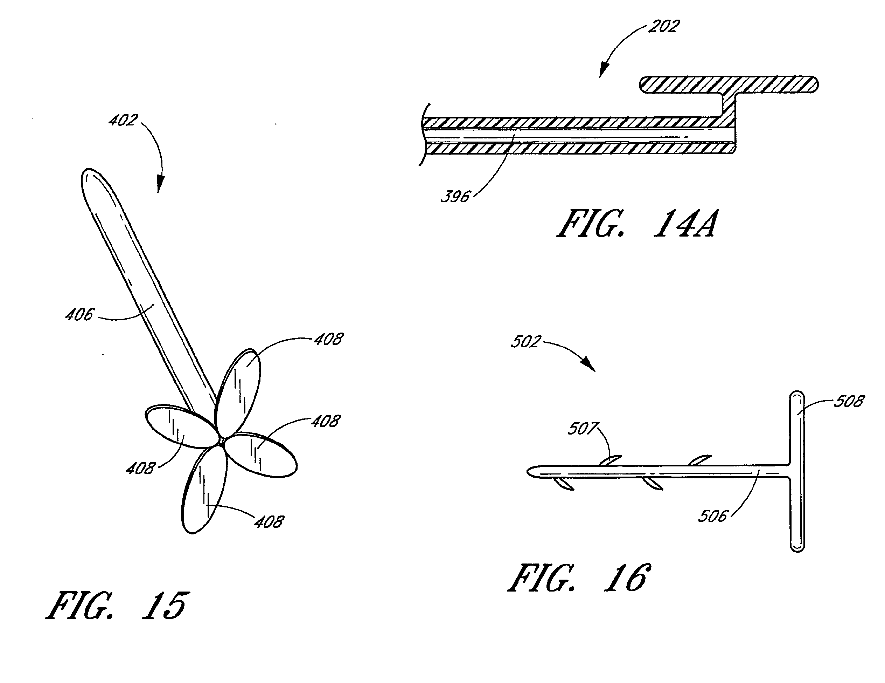

In accordance with another embodiment of the present invention, as shown in Figure 15, an occlusion device comprising an anchor element 402 may be provided. The anchor element 402 includes a plurality of occlusion retention portions 408 and an elongate body 406. In some embodiments, the retention portions 408 may be wire loops comprised of metal or polymer or other generally planar structures. The retention portions 408 may be attached to the elongate body by any of a variety of ways, such as soldering, welding, adhesively bonding, mechanically crimping or swaging, and the like. In some embodiments, the distal end of elongate body 406 may be provided with an opening (not shown) to facilitate attachment of retention portions 408 to the elongate body 406. In one embodiment, the anchor element 402 may be used with occlusion device 200 in place of the anchor element 202. Alternatively, anchor element 402 may be used alone as the occlusion device. In one embodiment, the retention portions 408 may be a plurality of petals. The actual number of retention portions 408 can vary. In one embodiment, the number of retention elements may be any value between about 2 and about 10. The actual number of retention elements may also vary significantly below 2 or above 10. The retention portions 408 provide additional retentional support, thereby preventing migration of the septum primum 306 away from the septum secundum 304.

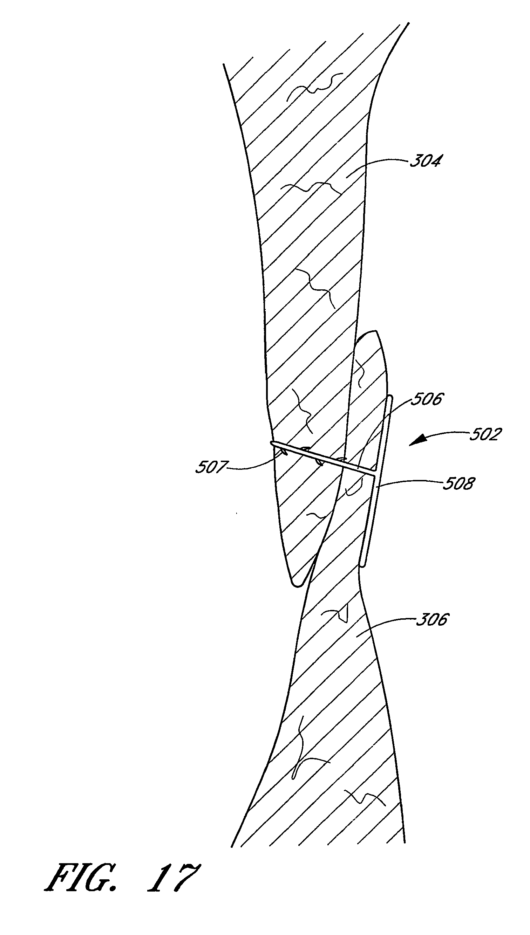

Alternatively, an occlusion device comprises an anchor element 502 having one or more retention structures 507 provided for retaining the device at the septal defect or other body region is shown in Figure 16. The anchor element 502 includes an elongate body 506 and a retaining portion 508. As shown, the anchor element 502 may be generally "T"-shaped. Although the anchor element 502 has been described as being "T"-shaped, it is also envisioned that the anchor element 502 may be other shapes, such as "I"-shaped, and the like.

In the illustrated embodiment, a plurality of barbs 507 or other retention structures are provided, for engaging adjacent tissue to retain the occlusion device 500 in its implanted position and to limit relative movement between the tissue and the occlusion device. Each of the barbs 507 is shown projecting generally radially outwardly from the longitudinal axis, toward the retaining portion 508.

The barbs 507 and corresponding anchor element 502 may be cut from a single ribbon, sheet or tube stock. Barbs 507 may be cut from elongate body 506, formed of tube stock. Retaining portion 508 may be attached to the elongate body 506 by any means, such as welding, adhesives, mechanical interlock and the like. Alternatively, each barb 507 may be laser cut from the anchor element 502. In some embodiments, the anchor element 502 may be molded from an engineering polymer. The anchor element 502 which carries the barbs 507 may be advanced from a low profile orientation in which each of the barbs 507 is generally parallel to the longitudinal axis, to an implanted orientation as illustrated, in which the barbs 507 are positioned radially outwardly from the longitudinal axis. In such an embodiment, the barbs 507 may be biased towards the enlarged orientation.

As described above, the illustrated retention structures 507 are in the form of barbs. Depending upon the embodiment, three or four or more barbs may be desired on the occlusion device. The barbs may be inclined in a proximal direction, a distal direction, or both to prevent distal movement, proximal movement, or both distal and proximal movement.

Any of a wide variety of structures may be utilized for retention structure 507, such as hooks, barbs, pins, sutures, adhesives, ingrowth surfaces and others which will be apparent to those of skill in the art in view of the disclosure herein. In one embodiment, the anchor element 502 may be used alone, while in other embodiments a retention element, such as retention element 204 shown in Figures 2C-E, may be used with anchor element 502. In some embodiments, where a retention element is used with the anchor element 502, the retention structures 507 can be configured to securely interlock with the retainer element in the vicinity of retainer element. For example, retention structures 507 may be positioned along elongate body 506 such that the axial thickness of the retention element 204 is slightly less than the axial separation of the retention structures 507. Alternatively, the retention structures 507 may mechanically interlock with the imbedded tines 216 of the retention element 204.

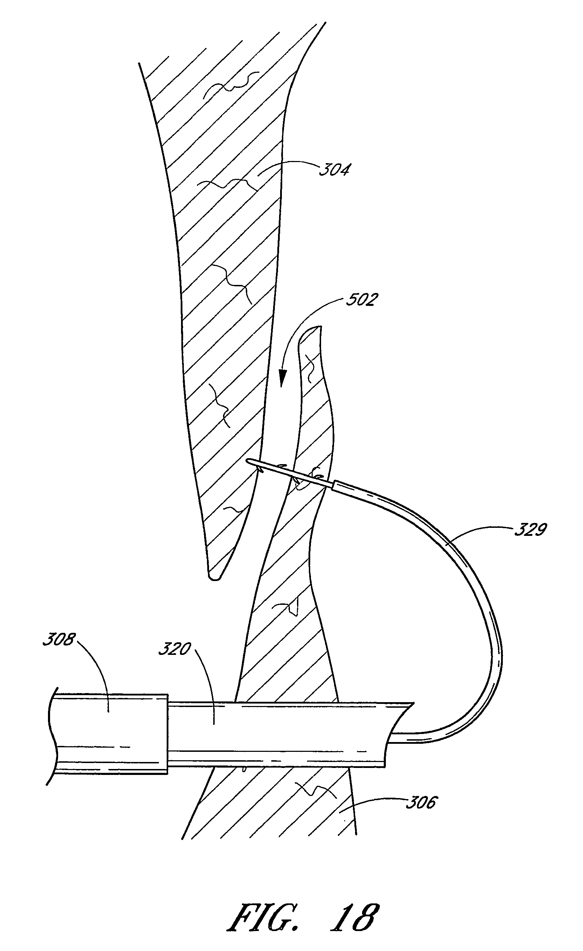

Figure 17 shows anchor element 502 deployed at a treatment site. The anchor element 502 may be delivered to the foramen ovale as shown in Figures 6-11. However, the actual method of delivery may vary. For example, the tissue retention structures 507 may eliminate the need for the retaining element 204 disclosed with reference to the occlusion device 200, because the barb orientation prevents movement of the anchor element 502 from the treatment site. Furthermore, anchor element 502 may be configured to penetrate the septum, thereby eliminating the need for a tissue piercing structure, as described with reference to the delivery system 300. For example, the elongate body 506 or anchor 508 may include a sharp or pointed end for piercing tissue at the treatment site.

Figure 18 shows an alternative delivery method, wherein an occlusion device may be delivered from the right atrium to the left atrium. The tissue piercing structure 320 is delivered to and passes through the septum primum 306. The actuator 329 may be actuated to deliver the occlusion device 500 to septum primum 306. The anchor element 502 is configured to puncture the septum primum 306 and the septum secundum 304. In one embodiment, the distal end of elongate member 506 is sharpened to puncture the septa. The tissue retention structures 507 desirably draw and retain the septum secundum 304 and septum primum 306 toward one another. By approaching the treatment site through the left atrium, it may be possible to better locate the treatment site. The actuator 329 may be a steerable catheter for anchor delivery. Alternatively, the actuator 329 may be made from a shape memory material having a preset curve. Alternatively, a steerable catheter or a catheter having a preset curve (not shown) may be delivered through the tissue piercing structure 320, while an actuator 329 is delivered through the catheter.

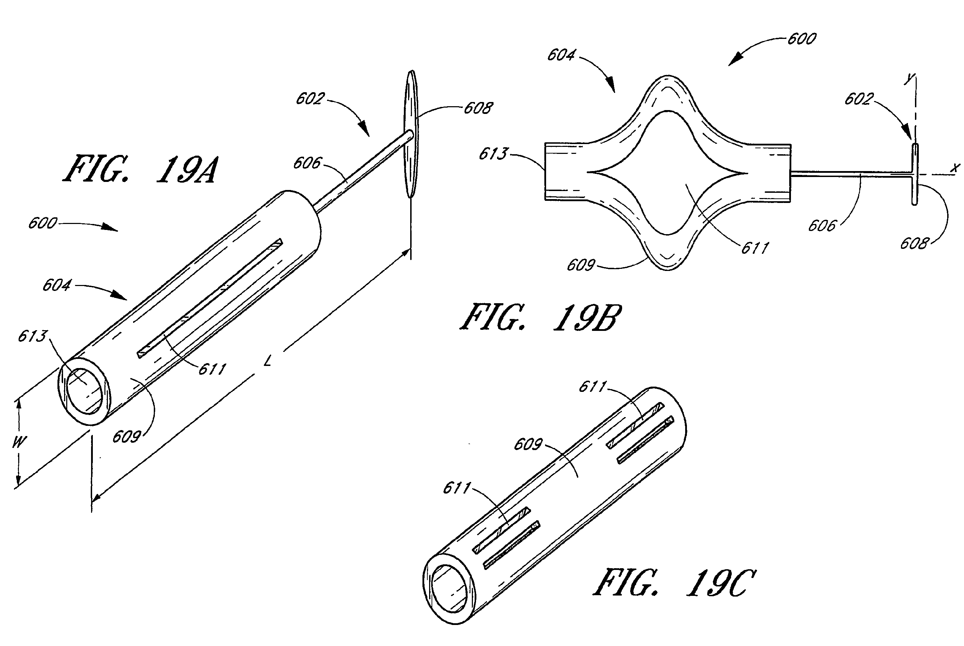

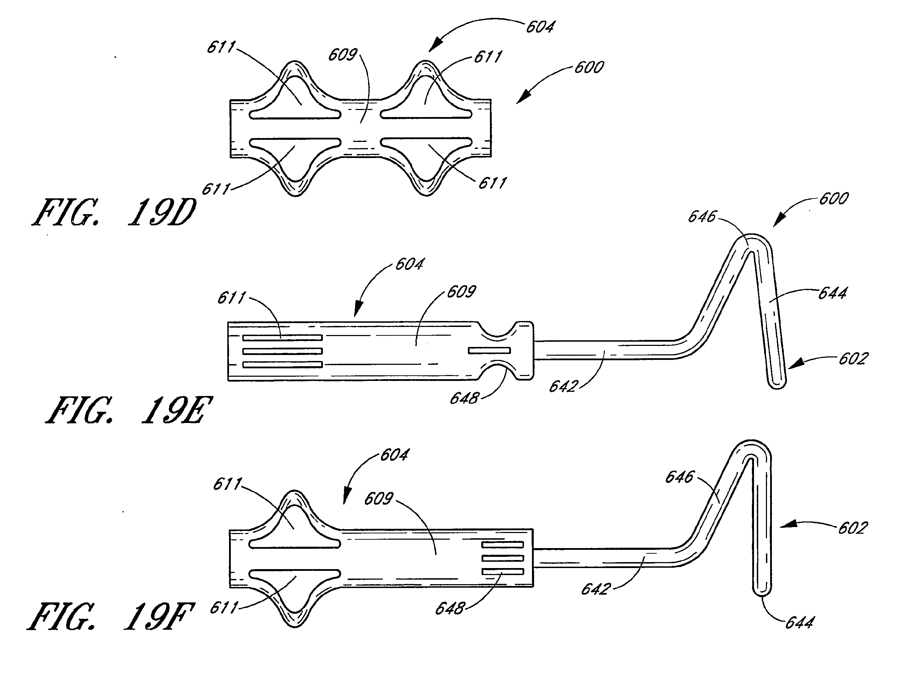

In another embodiment, as shown in Figures 19A and 19B, an occlusion device 600 having an anchor element 602 and a retention element 604 may be provided. The anchor element 602 has an elongate body portion 606 and a retaining portion 608. In one embodiment, the longitudinal axis x of the elongate body portion 606 is perpendicular to the longitudinal axis y of the retaining portion 608. In some embodiments, the angle between the longitudinal axis x of the elongate body portion 606 and the longitudinal axis y of the retaining portion 608 may vary. In some embodiments, the angle between the axis x and the axis y may be any value or range of values between about 15 and 165 degrees. In another embodiment, the angle value or range of values can vary below about 15 degrees and above about 165 degrees. The anchor element 602 may have a circular, rectangular or other-shaped cross-section, depending upon the manufacturing technique.

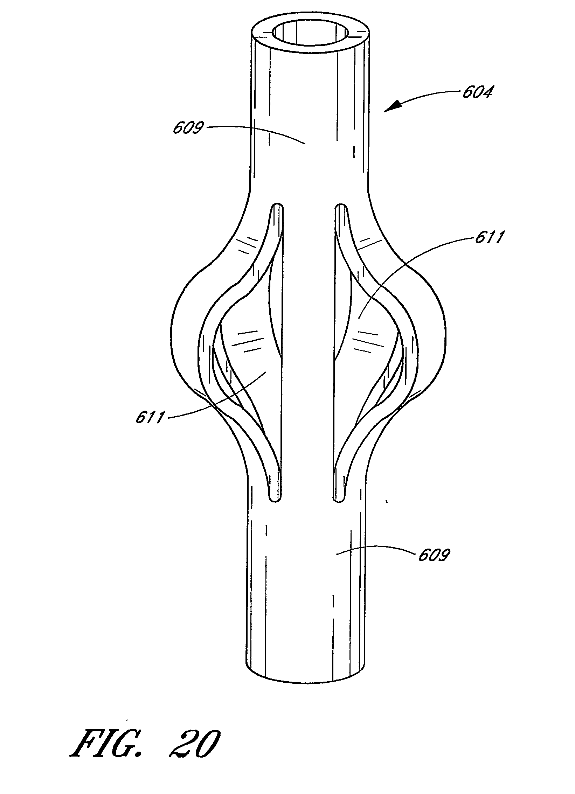

The retention element 604 has a tubular body 609 having at least two longitudinal openings 611, as shown in FIG. 19A (only one opening is shown). When the tubular body 609 is axially compressed, the region of the tubular body having the at least two longitudinal openings 611 expands radially, as shown in FIG. 19B. The retention element 604 may also include an attachment element 613 for releasably attaching the occlusion device 600 to a delivery device (not shown). In one embodiment, the attachment element 613 may be similar to the releasable attachment system described in Figure 12. Figure 20 shows a detailed perspective view of one embodiment of the retention element in an expanded state.

The anchor element 602 may be connected to the retention element 604 by any technique such as welding, brazing, interference fit, threaded connection, snap fit, crimping, and the like.

In one embodiment, the occlusion device 600 may be made from a medical plastic or a metal, such as stainless steel, Nitinol, Elgiloy®, or others which can be determined through routine experimentation by those of skill in the art. In anoth er embodiment, the occlusion member 600 may be made of a dissolvable suture material. The occlusion device 600 may also be biodegradable. It is also envisioned that other metallic or non-metallic biocompatible materials may be used to form occlusion device 600. The occlusion device 600 may be made from a shape memory material preset to its expanded shape, such that the occlusion device 600 self-expands. In one particularly preferred embodiment, the occlusion device 600 is made from Nitinol, a nickel-titanium alloy commonly used for shape memory and super-elastic medical device applications.

The elongate body portion 609 may have a circular, rectangular or other-shaped cross-section, depending upon the manufacturing technique. In one embodiment, a circular cross section is cut such as by known laser cutting techniques from tube stock. In one embodiment, the occlusion device 600 may be an integral structure, such as a single ribbon or wire, or an element cut from a tube stock.

Figure 19C shows another embodiment of occlusion device 600 having two radially enlargeable sections. The occlusion device includes an elongate body portion 609 having at least two longitudinal openings 611, each at or near the distal end and proximal end of the elongate body portion 609. Figure 19C shows an integral structure laser cut from Nitinol tube stock, and Figure 19D shows the structure of Figure 19C in an expanded state. When the tubular body 609 is axially compressed, the regions of the tubular body having the at least two longitudinal openings 611 expand radially. The septum secundum 304 and septum primum 306 may be positioned between the two radially enlargeable sections to secure the septum secundum 304 and septum primum 306 together.

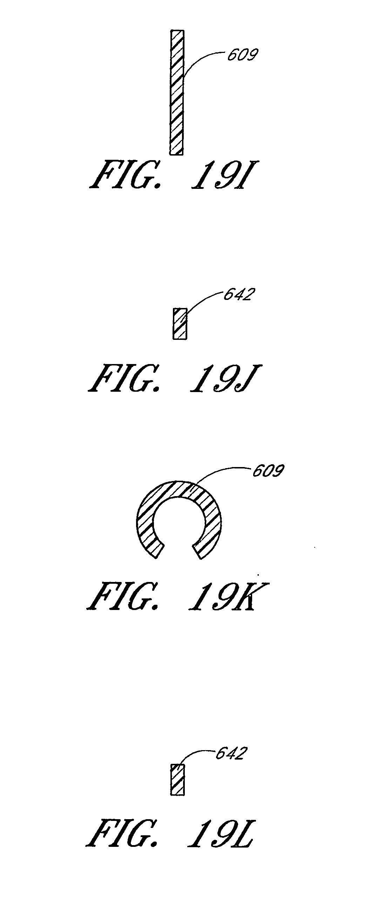

Figure 19E shows an occlusion device 600 having a retention element 604 and an anchor element 602 having an elongate body portion 642, a distal segment 644, and an intermediate portion 646 provided between the elongate body portion 642 and the distal segment 644. The intermediate segment 646 and distal segment 644 form a bend or hook element for securing the septum primum 306, as described below. The anchor element 602 is attached to tubular body 609 of retention element 604, the retention element 604 having at least two longitudinal slits 611. The angle between intermediate segment 646 and distal segment 644 may vary. In one embodiment, the angle between the intermediate segment 646 and distal segment 644 is about zero, such that the two segments together form a structure that approximates the crossbar of a "T"-shape. In one embodiment, the retention element 604 is a member laser cut from Nitinol tube stock, and crimped to the elongate body portion 642 of anchor element 602 at attachment point 648. The assembly may be heat set into the expanded shape shown in Figure 19F with the retention element 604 being normally expanded. The retention element, when drawn into a delivery device, may temporarily straighten out if engineered to not plastically deform when in the delivery configuration. The retention element self expands to the shape shown in Figure 19F, which may be characterized as a "T"-shape, when removed from the delivery configuration. When deployed, the occlusion device shown in Figure 19F is positioned such that the septum secundum 304 and septum primum 306 are secured between the radially expanded section 611 and the anchor element 602, as described below.

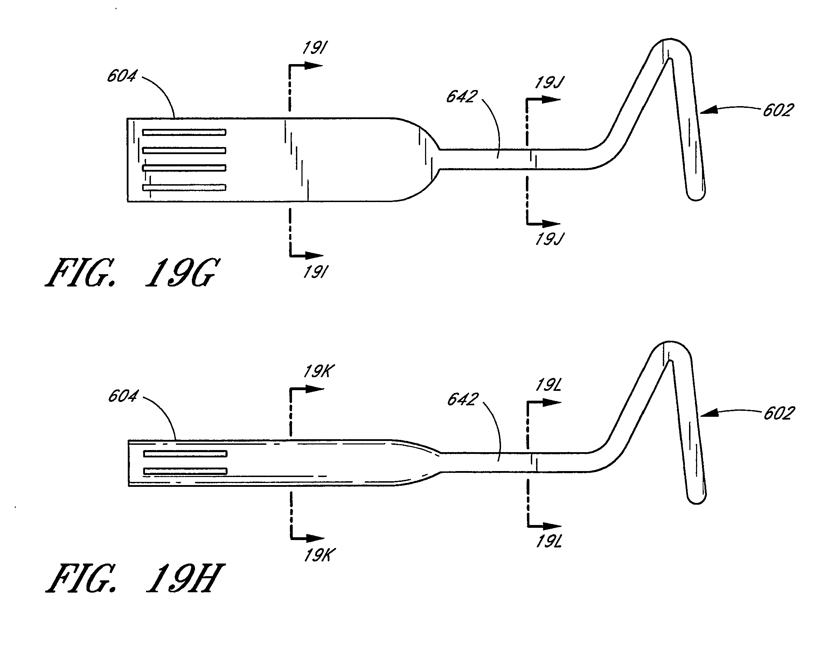

Figure 19G illustrates another embodiment of an occlusion device 600 formed from a sheet of Nitinol having been electrofonned or electroetched to form an unexpanded retention element 604, an anchor element 602, with an elongate body portion 642 therebetween. Cross-sections of this device are shown in Figures 191 and 19J. In Figure 19H, the sheet of Nitinol has been shape set to assume a substantially tubular configuration in the vicinity of the retention element 604. Cross-sections of this device are shown in Figures 19K and 19L. Further shape setting can establish an expanded retention element with self-expanding characteristics.

As shown in Figure 19A, the overall width W of the occlusion device 600 may be any value or range of values from about 10 mm to about 50 mm and, in one more preferred embodiment, is about 2.5 cm in its expanded state. In other embodiments, the overall width W of the occlusion device 600 may be significantly less than about 10 mm or greater than about 50 mm. The overall length L of the occlusion device 600 from the distal end to the proximal end in one embodiment may be any value or range of values from about 4 mm to about 20 mm and, in one more preferred embodiment, is about 8 mm in its expanded state. In other embodiments, the overall length L of the occlusion device 600 may be significantly less than about 4 mm or greater than about 20 mm. The occlusion device 600 is preferably small enough to fit into a percutaneous catheter in its compressed state. In one embodiment, the occlusion device in its compressed state is small enough to fit in a percutaneous catheter having a diameter from about 5 to about 12 Fr. In other embodiments, catheter having diameters of other sizes may be used. For example, catheters may be used having diameters of less than about 5 Fr. and greater than about 12 Fr.

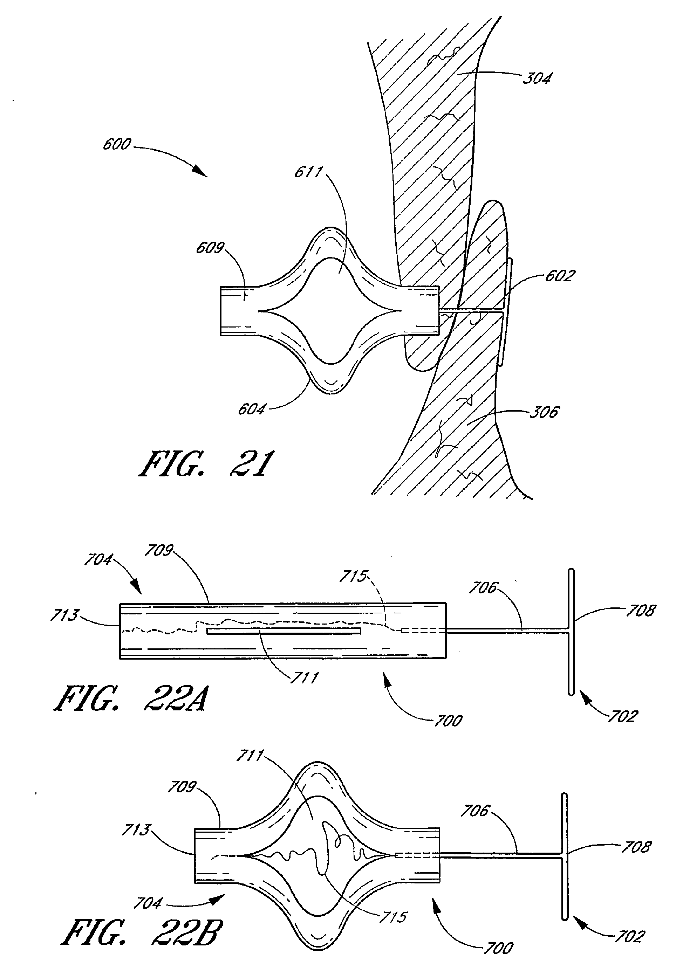

In use, a delivery device, such as delivery device 300 described herein, may be used to deliver the occlusion device 600 to a treatment site. The method of delivery of the delivery catheter described in FIGS. 6-11 may be generally used to deliver occlusion device 600 such as shown in FIG. 19A. Once the anchor element 602 is delivered to the treatment site, the retention element 604 may be expanded. In one embodiment, the retention element 604 self-expands to its expanded state when the retention element 604 is pushed out of the delivery catheter (or a tissue piercing structure as described above, if used). In one embodiment, the retention element 604 is delivered by compressing the distal and proximal ends of the retention element toward one another. The compression may be accomplished with a pull wire, push wire, torque rod, and the like. FIG. 21 shows the occlusion device 600 delivered at a treatment site.

Occlusion device 600 is particularly advantageous because the self-expanding retention element 604 allows the device to be engineered to accommodate a wide range of septal wall thicknesses, thus reducing the need to pre-measure wall thickness and choose an appropriately sized occluder. Also, retention element 604 provides a long-acting clamping force between the retaining portion 608 and retaining elements 604. The long-acting force assists with tissue growth and/or scarring between the septum secundum 304 and septum primum 306 by maintaining close apposition between the surfaces, particularly in the vicinity of elongate body portion 642 where tissue penetration has occurred and a healing response is expected.

In another embodiment, as shown in Figures 22A and 22B, an occlusion device 700 having an anchor element 702 and a retention element 704 may be provided. The anchor element 702 has an elongate body portion 706 and a retaining portion 708. In one embodiment, the longitudinal axis x of the elongate body portion 706 is perpendicular to the longitudinal axis y of the retaining portion 708. In some embodiments, the angle between the longitudinal axis x of the elongate body portion 706 and the longitudinal axis y of the retaining portion 708 may vary. In some embodiments, the angle between the axis x and the axis y may be any value or range of values between about 15 and 165 degrees. In another embodiment, the angle value or range of values can vary below about 15 degrees and above about 165 degrees. The anchor element 702 may have a circular, rectangular, or other shaped cross-section, depending upon the manufacturing technique.

The retention element 704 has tubular body 709 having at least two longitudinal openings 711, as shown in FIG. 22A. In an embodiment having two openings as shown in FIG. 22A, a second opening is provided at approximately 180 degrees from the opening shown. However, it is envisioned that more than two openings may be used. In some embodiments, each of the at least two openings are positioned equiangularly. When the tubular body 709 is axially compressed, the region of the tubular body having the at least two longitudinal openings 711 expands radially, as shown in FIG. 22B. The retention element 704 may also include an attachment element 713 for releasably attaching the occlusion device 700 to a delivery device (not shown). The retention element 704 also includes a tissue ingrowth element 715.

It may be desirable to enable tissue ingrowth in the vicinity of the occlusion device 700, such that the device can be scarred over or incorporated into the surrounding tissue. Furthermore, it may be desirable to stimulate scarring of the septum secundum 304 and septum primum 306 such that they become grown into permanent attachment with one another, thereby reducing the risk of emboli passing through the PFO into the arterial circulation.

A Dacron, polyester, or other tissue growth prompting material may be used with the implant. In one embodiment, the tissue ingrowth element 715 may be a thread, a strip, a series of stands or the like, such that the tissue ingrowth element 715 fits within or near the vicinity of occlusion device 700 but does not interfere with delivery or implantation of the device.

As an alternative to or in combination with the tissue ingrowth element, the occlusion device 700 may be coated with a thin layer of a tissue ingrowth material, such as collagen, polyester, ceramic, and the like. In one embodiment, at least a portion of occlusion device 700 may be coated with a fabric comprising the tissue ingrowth material. In one embodiment, the occlusion device 700 may comprise a coating over at least a portion of the anchor element 702, the elongate body portion 706 and/or the retention element 704.

In one embodiment, the occlusion device may be made of polyester, having a roughened surface, thereby achieving tissue ingrowth post-implant.

In one embodiment, the occlusion device 700 may be made from a medical plastic or a metal, such as stainless steel, Nitinol, Elgiloy®, or others which can be determined through routine experimentation by those of skill in the art. In another embodiment, the occlusion member 700 may be made of a dissolvable suture material. The occlusion device 700 may also be biodegradable. It is also envisioned that other metallic or non-metallic biocompatible materials may be used to form occlusion device 700.

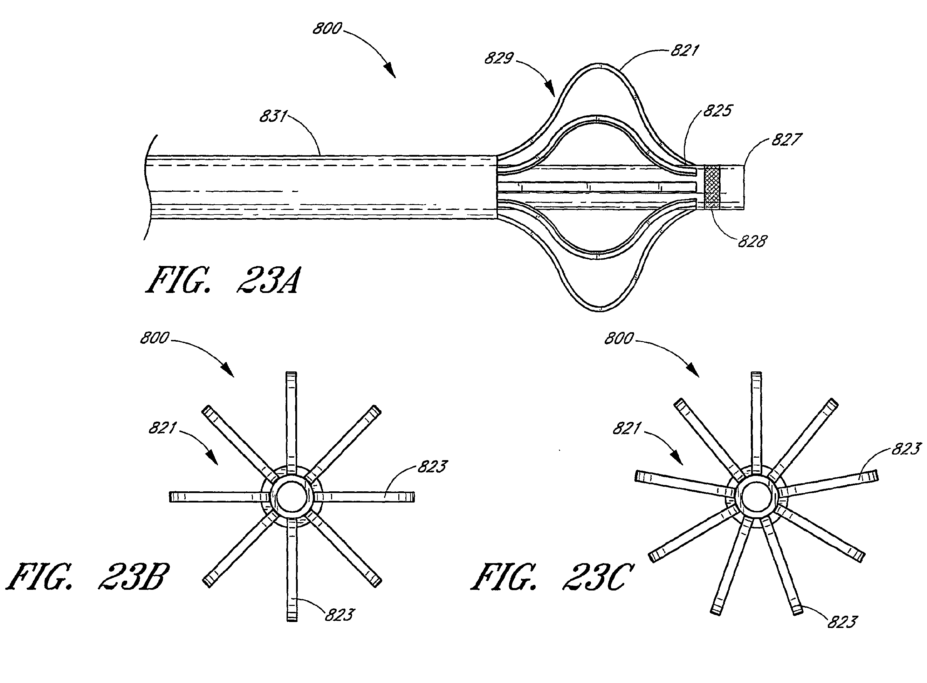

In another embodiment, as shown in FIGs. 23A and 23B, a delivery device 800 having a stabilization element 821 may be provided for stabilizing a delivery catheter in the left or right atrium. Stabilization of the delivery catheter may improve location of a treatment site. The stabilization element 821 may be collapsed to a transluminal delivery configuration and expanded to a stabilizing configuration. In one embodiment, the stabilization elements 821 may be expanded to a stabilizing configuration in the right atrium of the heart. In another embodiment, the stabilization elements 821 may be expanded to a stabilizing configuration in the left atrium of the heart. In still other embodiments, the stabilization elements 821 may be expanded in another chamber of the heart or passageway, such as a ventrical or vessel. In some embodiments, the stabilization elements 821 are expanded to contact the inner wall of the left atrium and the distal end of the sheath 827 is relatively constantly positioned in relation to a treatment location, such as a patent foramen ovale.

In one embodiment, the stabilization element 821 comprises a plurality of self-expanding struts 823. Any number of struts may be used. In some embodiments, at least three struts are used. Struts 823 may be attached at a proximal end to catheter body 831 and at a distal end to sheath 827. The struts 823 may be symmetrically arranged around sheath 827 as shown in Figure 23B. In another embodiment, the struts may be asymmetrically arranged around sheath 827, as shown in Figure 23C. The particular asymmetrical orientation may vary from the embodiment shown in Figure 23C. In some embodiments, the stabilization element 821 may be made from braided strands. In other embodiments, the stabilization element 821 may be a balloon with an inflation tube affixed thereto, as known to those of skill in the art.

Sheath 827 may also include at least one radiopaque marker band 828. Radiopaque marker 828 may be made from platinum, gold, iridium or the like. In some embodiments, the radiopaque marker may include a region of radiopaque filler in the sheath material. The filler may be made from barium sulfate, bismuth subcarbonate, tungsten powder, or other materials as are commonly employed in the art. Sheath 827 and/or catheter body 831 may also be reinforced with metallic braids or other materials as is commonly known in the art for improving torsional stiffness to the tubular shafts.

In one embodiment, the stabilization element 821 may be made from a medical plastic or a metal, such as stainless steel, Nitinol, Elgiloy®, or others which can be determined through routine experimentation by those of skill in the art. In another embodiment, the stabilization element 821 may be made of a dissolvable suture material. The stabilization element 821 may also be biodegradable. It is also envisioned that other metallic or non-metallic biocompatible materials may be used to form stabilization element 821.

In some embodiments, the inner diameter of the delivery device 300, described above, is sized to permit passage of the delivery device 800. In one embodiment, the device may delivered from the left atrium to the right atrium, and used with the delivery catheter as discussed with reference to Figure 18.

In use, the stabilization element 821 may self-expand. In another embodiment, the stabilization element 821 may expand by compressing the ends of the stabilization element 821. For example, the distal end 825 of the stabilization element 821 may be attached to a sheath 827 at the distal end of the delivery device 800. The distal end 825 may be welded or bonded or crimped or otherwise affixed to the sheath 827. The proximal end 829 of the stabilization element 821 may similarly be attached to the catheter body 831. By moving the sheath or catheter relative to one other, the stabilization element 821 expands. In an alternative embodiment, self-expanding stabilization elements 821 are attached at both ends to the distal portion of a catheter body, and an outer sleeve slideably receives the catheter body. Proximally retracting the sleeve will allow the stabilizer elements to self-expand. Stabilization elements of this type can also be incorporated onto the device shown in Figure 18.

After the stabilization element 821 is expanded, a delivery device, such as delivery device 300, may be passed through the delivery device 800 for delivering an occlusion device to a treatment site. In some embodiments, sheath 827 is sized to at least partially, slideably receive delivery system 300 or the delivery system as disclosed with reference to Figure 18.

In some embodiments, the delivery device 800 may be percutaneously delivered to the right atrium using the catheter techniques described herein. Stabilization element may be expanded and collapsed, rotated, and re-expanded until the tip of the delivery device 800 is suitably located near to the treatment site. A radiopaque marker or radiopaque dye injection may be used to visualize the delivery device 800 at the treatment site. An occlusion device delivery system, such as device 300, may slideably pass through the delivery device 800 for delivering an occlusion device to a treatment site.

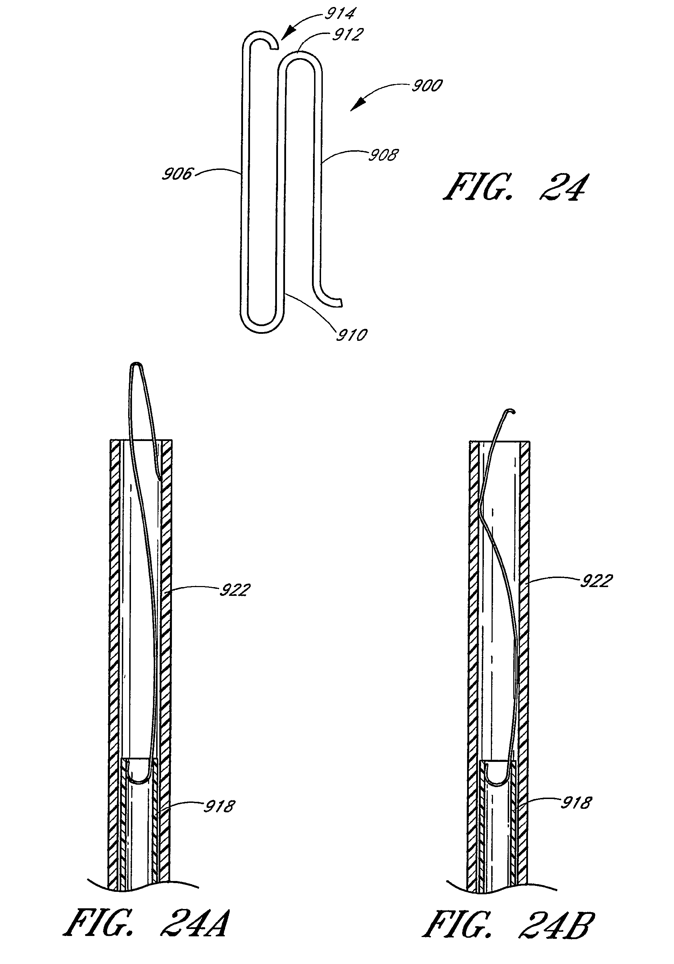

In accordance with another embodiment of the present invention, as shown in Figures 24-31, an occlusion device comprising a spring clip may be provided to secure the defect. The occlusion device preferably comprises a wire shaped to form a clip. In some embodiments, the wire may be Nitinol. Although the device is shown having a clip shape, it is envisioned that a number of variations of this shape can be utilized to provide the same results. For example, a bend may be provided in the device to aid in closure. Also, annular, non-circular or round shapes may be used.

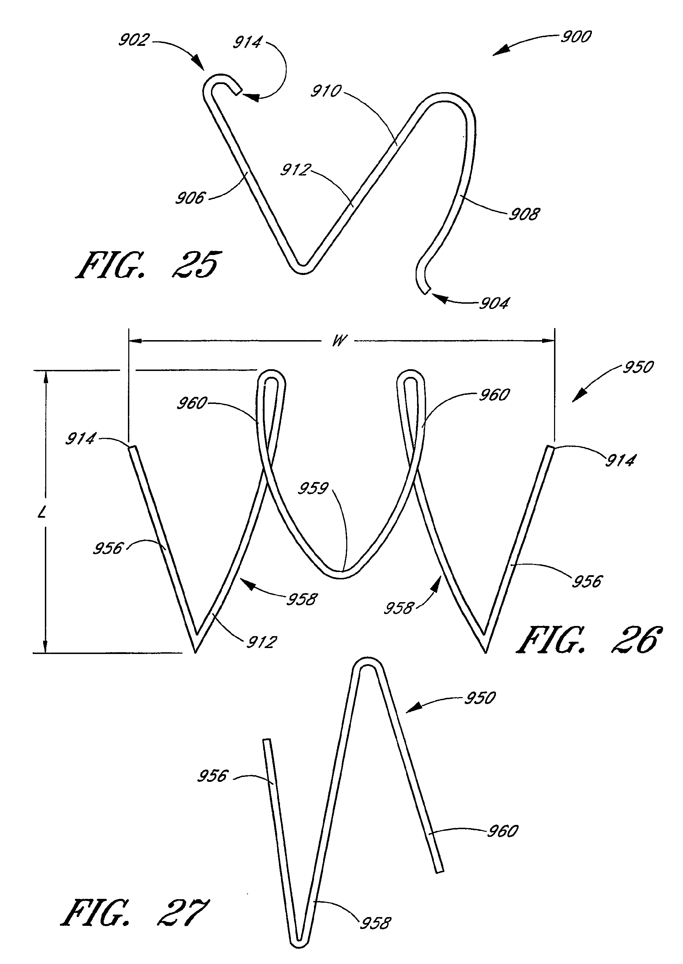

As shown in Figure 25, the occlusion device 900 has a proximal end 902 and a distal end 904. The designation "proximal" indicates the position of the device closest to the operator when the device is within the deployment catheter and is used for description purposes only and is not intended to designate a preferred or required orientation or arrangement.

As shown in Figures 24 and 25, the occlusion device 900 generally has three sections: a proximal segment 906, a distal segment 908, and an intermediate segment or occlusion segment 910. The segments, 906, 908, 910 are formed from wire 912. The segments 906, 908, 910 form an integral structure for occluding a patent foramen ovale. The occlusion device 900 is also preferably provided with a detachment element 914 at its proximal end 902. Alternatively, the proximal end 902 of the device 900 may be provided with a threaded aperture through which a delivery core is threadably engaged, as described with respect to Figure 17.

The intermediate segment 910 is positioned between the septum secundum 304 and the septum primum 306 to close the patent foramen ovale 302, as shown in Figure 29A and 29B. The proximal segment 906 is positioned in the right atrium, while the distal segment 908 is positioned in the left atrium.

In one embodiment, occlusion device 900 may be delivered to the patent foramen ovale 302 in its expanded state (see Figure 29A), and then compressed to secure the patent foramen ovale 302 in a closed position (see Figure 29B). In one embodiment, the occlusion device 900 may be biased to spring to the closed position upon delivery. In one embodiment, the occlusion device 900 may be compressed under positive force to close the occlusion device 900.

The occlusion device 900 is designed to be implanted using a delivery catheter. The device may be designed to be delivered in an elongated state or remain in a collapsed state while in the catheter. As shown in Figures 24A, 24B, all or a portion of the Nitinol wire clip may be straightened for delivering through a catheter 922. In this embodiment, the Nitinol wire clip has preferably been heat treated to its clip shape such that this shape is remembered after exiting the delivery catheter. The detachment element 914 on the occlusion device can be releasably attached to a push rod 918, which is adapted to push the occlusion device out of the end of the catheter. Upon delivery to the patent foramen ovale, the device 900 is positioned and expanded to occlude the patent foramen ovale. Figure 25 shows the device in a partially expanded state. The procedure for placing the occlusion device and delivery device will be described in further detail hereinafter.

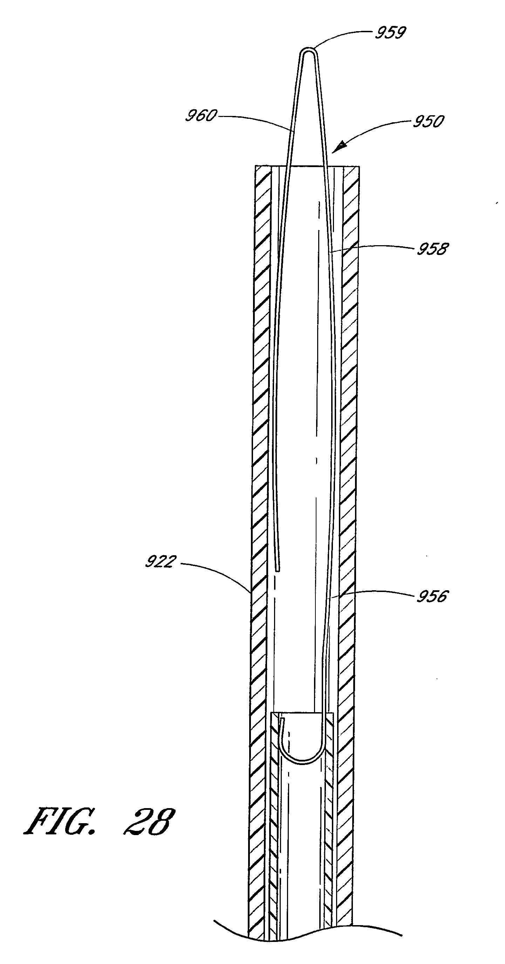

Figures 26 and 27 show an alternative embodiment of a clip-like occlusion device. Figure 26 is a schematic view of occlusion device 950. Figure 27 is a side view showing occlusion device 950. Occlusion device 950 generally has three sections: proximal segments 956, a distal segment 960, and intermediate segments or occlusion segments 958. The segments, 956, 958, 960 are formed from wire 912. The segments 956, 958, 960 form an integral structure for occluding a patent foramen ovale. The occlusion device 950 is also preferably provided with detachment elements provided at the proximal end of the proximal segments 956, as previously discussed with respect to other embodiments. Alternatively, the device 950 is provided with at least one threaded aperture through which a delivery core is threadably engaged. The distal segments 958 are joined at an apex 959.

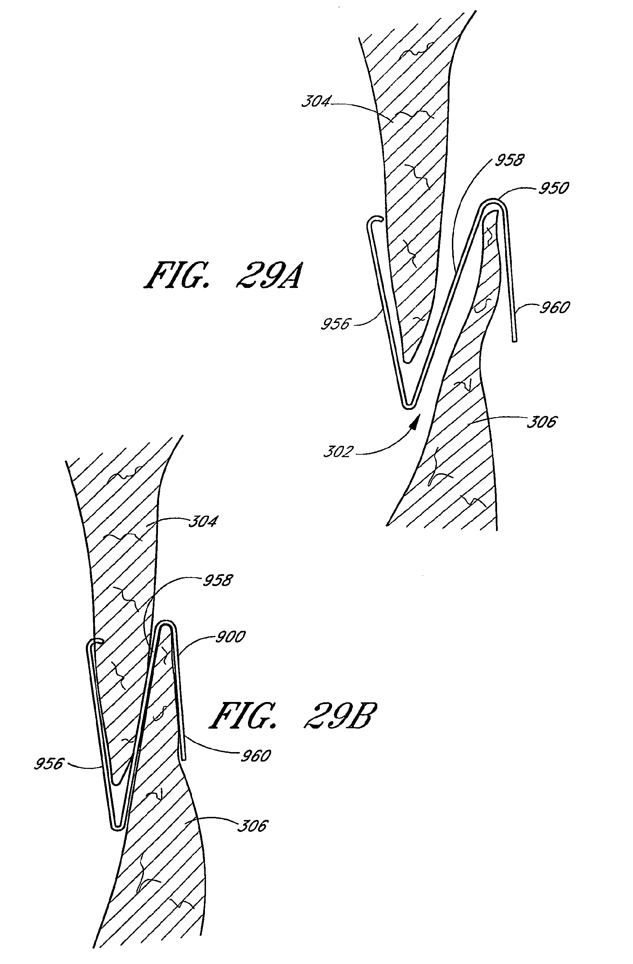

Figure 28 shows a delivery device 922 for delivering the occlusion device 950. Occlusion device 950 is elongated and positioned in catheter 922, such that apex 959 is distal to proximal segments 956, intermediate segments 958, and distal segment 960. In some embodiments, the device may be biased to self-expand upon delivery at the patent foramen ovale. In other embodiments, the device may be compressed upon delivery at the patent foramen ovale. The occlusion device 950 may be releasably attached to the push rod 918 using any suitable mechanism, such as a suture line, threading, etc. In some embodiments, occlusion device 950 may be attached to a push rod 918, using detachment elements similar to those described with reference to Figures 12 and 13.

As shown in Figure 29A, the intermediate segments 958 are positioned between the septum secundum 304 and septum primum 306, the distal segment 960 is positioned against the septum primum 306, and the proximal segments are positioned against the septum secundum 304 to close the patent foramen ovale 302, as shown in Figure 29B.

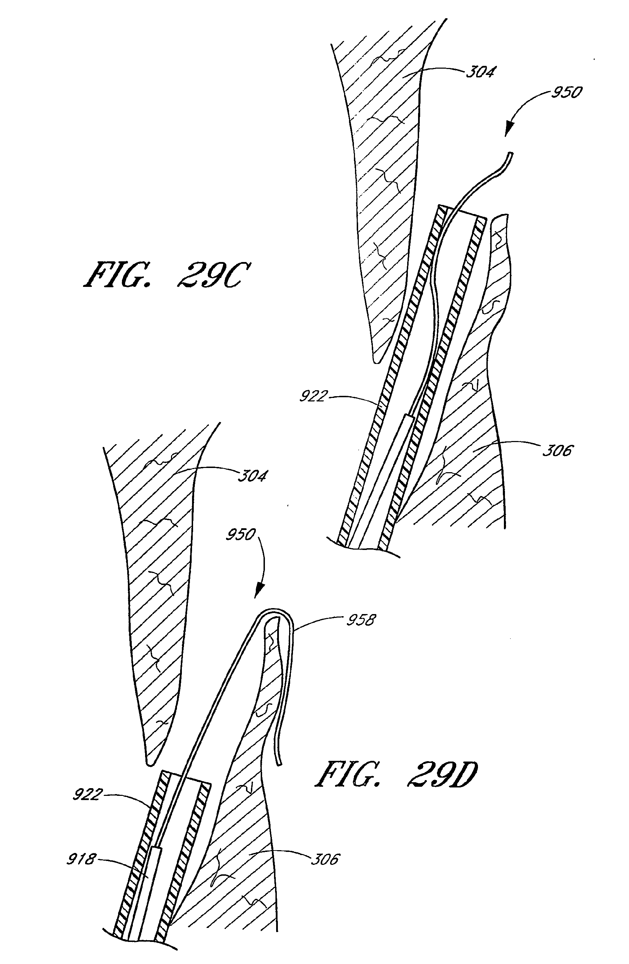

In one embodiment, occlusion device 950 may be delivered to the patent foramen ovale 302 in an elongated state (see Figure 29C), wherein the occlusion device 950 is essentially elongated. The occlusion device 950 is then manipulated to secure the patent foramen ovale 302 in a closed position as the wire is advanced from the delivery device 922 (see Figure 29D). In one embodiment, the occlusion device 950 may be biased to spring to the closed position upon delivery. Alternatively, the occlusion device 950 may be compressed under positive force to close the occlusion device 950.

Preferably, the wire 912 comprises a metal such as stainless steel, Nitinol, Elgiloy®, or others which can be determined through routine experimentation by those of skill in the art. The wire may also be biodegradable. Wires having a circular, rectangular or other cross-section may be utilized depending upon the manufacturing technique. In one embodiment, a circular cross section wire is cut such as by known laser cutting techniques from tube stock. The occlusion device is preferably an integral structure, such as a single ribbon or wire, or element cut from a tube or sheet stock. It is also envisioned that other metallic or non-metallic biocompatible materials may be used to form wire 912. In one embodiment, the wire 912 may be a shape memory material.

With reference to Figure 26, the device 900, 950 has an expanded width W having any value or range of values from about 0.127mm (0.005 in) to about 9.53mm (0.375 in), and, in one more preferred embodiment, about 6.35 (0.25 in). In some embodiments, the device 900, 950 may have an expanded width W of significantly less than about 0.127 mm (0.005 in) or greater than about 9.53 mm (0.375 in). The overall length L of the occlusion device 900, 950 from the distal end 904 to the proximal end 902 (when straightened) in one embodiment is any value or range of values from about (15.2 to 63.5 mm) (0.6 to 2.5 inches), and the folded (as implanted length) is about (5.08 to 19.1mm) (0.2 to 0.75 inches). In some embodiments, the overall length L when straightened may be significantly less than about (15.2 mm) (0.6 in) or greater than about (63.5 mm) (2.5 inches), and the implanted length may be significantly less than about (5.08 mm) (0.2 in) or greater than about (19.1 mm) (0.75 in). In some In some embodiments, the wire has a diameter of any value or range of values between (0.127-1.02mm) (0.005-0.0040 in), and, in one some preferred embodiments, any value or range of values between (0.203-0.508mm) 0.008-0.020 in). In some embodiments, the wire diameter may be significantly less than about 0.127mm (0.005 in) or greater than about 0.508 mm (0.020 in).

In some embodiments, radiopaque markers may be provided on the occlusion device 900, 950 to aid in placement at the treatment site. In some embodiments, the radiopaque markers are crimped on to the occlusion device. In one embodiment, the radiopaque markers are tubular bands crimped on to the occlusion device. In some embodiments, the radiopaque markers are coatings applied to the device or core wires within the wire 912. In some embodiments, the radiopaque markers may be platinum or iridium, and the like.

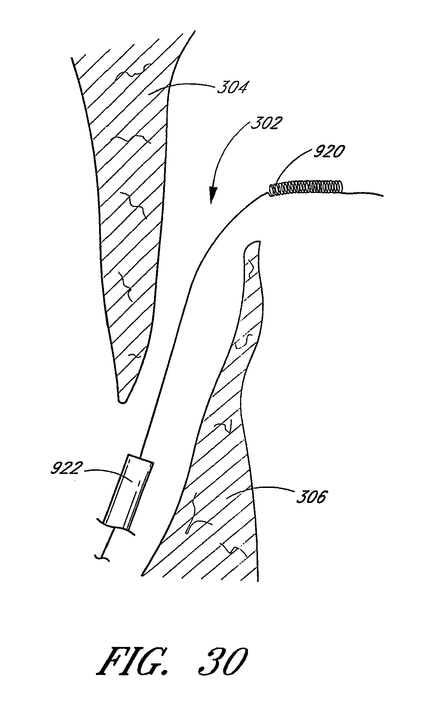

With reference to Figure 30, a guide wire 920 may be delivered between the septum secundum 304 and septum primum 306. A delivery device 922 is then delivered over the guide wire 920 to a septal defect 302. The delivery device 922 is preferably advanced between the septum secundum 304 and septum primum 306. The guide wire 920 may be removed. The occlusion device 900, 950 is delivered to the patent foramen ovale, by advancing at least a portion of the occlusion device 900, 950 out of the delivery device 922, as shown in Figure 29C. Alternatively, occlusion device 900, 950 may be delivered such that a portion 908, 958, 959, and/or 960 of the occlusion device extends out of the delivery device 922, thereby eliminating or reducing advancement of the occlusion device out of the delivery device 922. Further advancement of occlusion device 900, 950 out of catheter 922 causes distal segment 908, 960 to return to a shape set shape in contact with septum primum 306, as shown in Figure 29D. In one embodiment, by pulling the occlusion device 900, 950 and delivery device 922 proximally, the distal segment 908, 960 of the occlusion device 900 is secured over the septum primum 306, as shown in Figures 29A, 29B, and 29D. The occlusion device 900, 950 is released from the delivery device 922 by retracting the delivery catheter while holding the push rod 918 stationary and releasing push rod 918 from detachable element 914, such that proximal segment 906, 956 returns to a pre-set shape and becomes secured over the septum secundum 304. Alternatively, the delivery device 922 is removed from occlusion device 900 after delivery of the occlusion device 900 to a final position wherein proximal segment 906, 956 is in contact with the septum secundum 304 by detachment of pull rod 918 from detachment element 914.

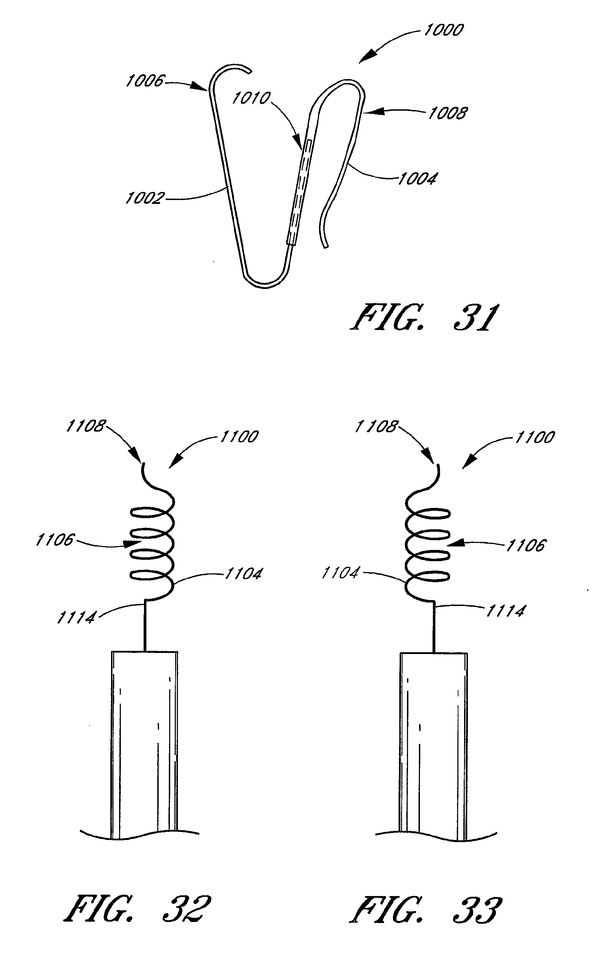

An adjustable occlusion device 1000 may be provided for adjusting to the specific anatomy at the treatment site, as shown in Figure 31. The adjustable occlusion device 1000 may include a first element 1002 and a second element 1004 which may be adjustable relative to one another. The first element 1002 and the second element 1004 combine to form three sections: a proximal segment 1006, a distal segment 1008, and an intermediate segment or occlusion segment 1010. As shown in Figure 31, the adjustable aspects of the device are preferably provided at the occlusion segment 1010, where the first element 1002 and second element 1004 join together. In one embodiment, the first element 1002 and second element 1004 may be telescoping. In one embodiment, the adjustable elements may include locking elements or tines (not shown) to lock the first element and second element in place at a desired adjusted location.

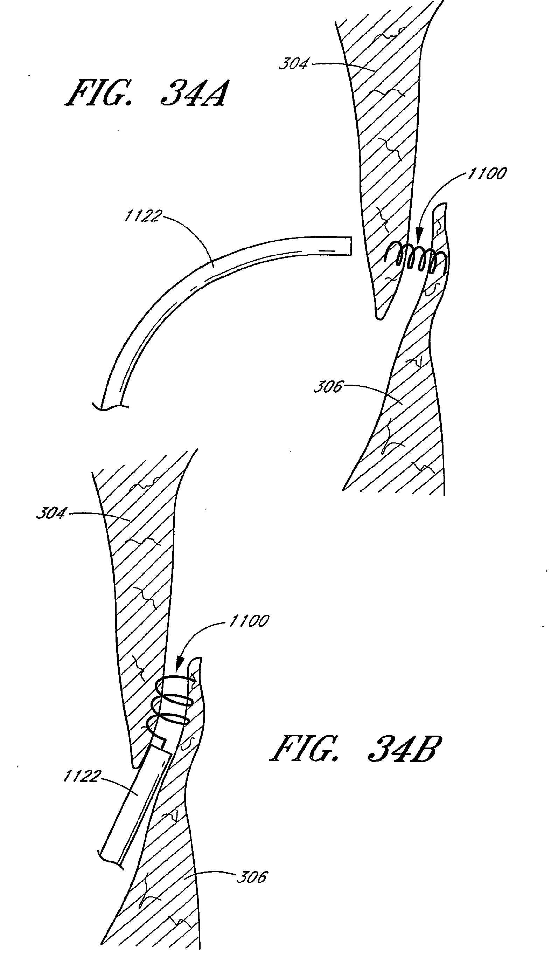

In accordance with another embodiment of the present invention, an occlusion device 1100 comprising a rotatable retention element may be provided. In one embodiment, rotatable retention element 1102 may have a left-handed threading, as shown in Figure 32. In one embodiment, rotatable retention element 1104 may have a right-handed threading, as shown in Figure 33. In some embodiments, the rotatable retention elements 1102, 1104 may be a coil 1106 having a pointed or sharpened end 1108 to penetrate tissue. The device 1100 may have a detachable element 1114 provided at the proximal end of the coil 1106. In some embodiments, the coil 1106 may have a variable pitch or more than one pitch.

In one embodiment, the occlusion device 1100 may be made from a medical plastic or a metal, such as stainless steel, Nitinol, Elgiloy®, or others which can be determined through routine experimentation by those of skill in the art. In another embodiment, the occlusion member 1100 may be made of a dissolvable suture material. The occlusion device 1100 may also be biodegradable. It is also envisioned that other metallic or non-metallic biocompatible materials may be used to form occlusion device 1100.

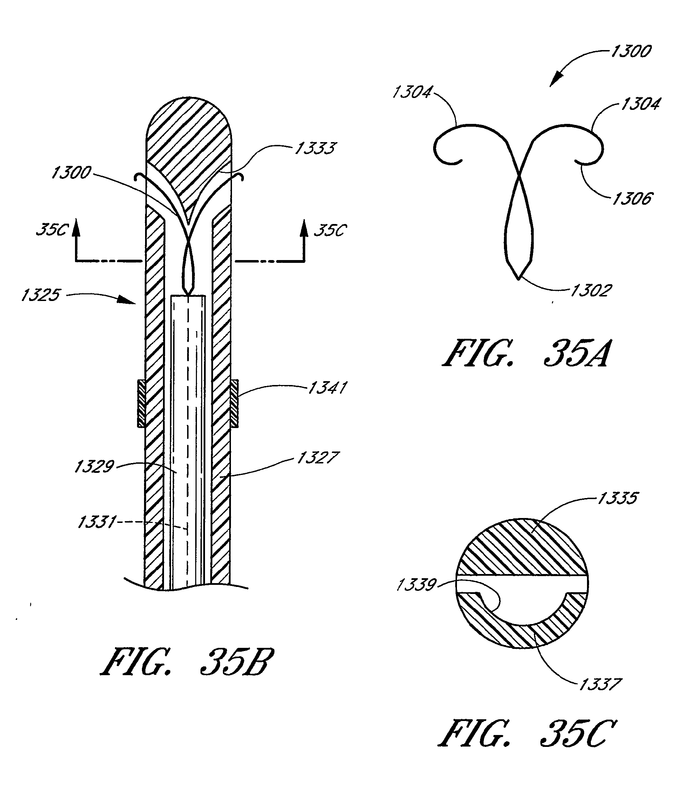

Figures 34A and 34B show occlusion device 1100 implanted at the foramen ovale. The occlusion device 1100 may be delivered to the foramen ovale using an occlusion device delivery system 1122 comprising a catheter having a rotatable actuator. The occlusion device 1100 may be rotated through the septum secundum 304 and septum primum 306, drawing the septum together to close the patent foramen ovale 302, as shown in Figure 34A. In some embodiments, occlusion device 1100 may have a coil 1106 with closer coil spacing near the proximal end of the device than near the distal end of the device (not shown), so that rotation of the coil 1106 tends to draw the septa together. Although the occlusion device 1100 is shown delivered from the right atrium, it may alternatively be delivered from the left atrium. In one embodiment, the occlusion device 1100 may be rotated through the septum secundum 304 and septum primum 306 from between the septum 304, 306, as shown in Figure 34B.

In another embodiment, an occlusion device may be delivered from between the septum secundum 304 and septum primum 306, and pull the septa together. In some embodiments, the occlusion device may include a single loop having sharp ends and retention elements, as described below. The loop may be pushed axially through a catheter to transversely engage the septa.

Referring to Figure 35A, an occlusion device 1300 having an apex 1302 and at least two loops 1304 is provided. The distal end of loop 1304 may be sharpened to penetrate tissue. The loop 1304 may include a retention element 1306. In some embodiments, the retention element 1306 is a barb configured to resist withdrawal from a septum once the barb has engaged the septum.

The occlusion device 1300 may be made from a medical plastic or a metal, such as stainless steel, Nitinol, Elgiloy®, or others which can be determined through routine experimentation by those of skill in the art. In some embodiments, the device may be biased to self-expand upon delivery at the patent foramen ovale. In another embodiment, the occlusion device 1300 may be made of a dissolvable suture material. In some embodiments, the occlusion device 1300 may also be biodegradable. It is also envisioned that other metallic or non-metallic materials may be used to form occlusion device 1300.

In some embodiments, the occlusion device 1300 may be heat-treated to its "clip" shape, such that the occlusion device is biased to expand to the "clip" shape when it exits the delivery catheter.

As shown in Figure 35B, all or a portion of the occlusion device 1300 may be generally straightened for delivering the device through a catheter 1325. The delivery system 1325 may be used to deliver occlusion device 1300 to a treatment location such as a patent foramen ovale. The delivery system 1325 contains an outer catheter 1327, an inner push tube 1329, and a thread 1331. The delivery system 1325 may also contain channels 1333 that can be used to guide loops 1304 in a direction transverse to the longitudinal axis of catheter 1327.

The occlusion device 1300 may be releasably attached to the push rod 1329 using any suitable mechanism, such as a suture line, threading, and the like. In some embodiments, the occlusion device 1300 may be attached to push rod 1329 using detachment elements similar to those described with reference to Figures 12 and 13. Upon delivery to the patent foramen ovale, the occlusion device 1300 is advanced out of catheter 1325 to draw the septum secundum 304 and septum primum 306 in close apposition.

Catheter 1327 may be manufactured in accordance with any of a variety of techniques. In one embodiment, the catheter 1327 may be extruded from any of a variety of materials, such as HDPE, PEBAX® , nylon and PEEK^{™}. In some embodiments, at least a portion of or all of the length of the catheter body may comprise a spring coil, solid walled hypodermic needle or other metal tubing, or a braided reinforced wall, as are known in the art. The spring coil, tubing, braided reinforcement, or other structures may be encapsulated with thermoset polymers such as polyimide and the like or with thermoplastic polymers such as PEBAX® and the like.

The push tube 1329 desirably has good column strength. Push tube 1329 may be formed from any of a variety of ways, such as a spring coil, solid walled hypodermic needle or other metal tubing, or a braided reinforced wall, as are known in the art. The spring coil, tubing, braided reinforcement, or other structures may be encapsulated with thermoset polymers such as polyimide or thermoplastic polymers such as PEEK^{™}, and the like.

The thread 1331 may be manufactured from a variety of high strength flexible materials such as metal wire or cable, Kevlar, polyester thread, oriented ultra-high molecular weight polyethylene, and the like.

Figure 35C shows a cross-section of catheter 1327. Catheter 1327 includes a first half 1335 and second half 1337. First half 1335 slides axially relative to second half 1337. Second half 1337 may contain channels 1333 and a lumen 1339 for push tube 1329. First half 1335 and second half 1337 may be held together by one or more collars 1341 (see Figure 36B) that can be used to guide loops 1304 in a direction transverse to the longitudinal axis of catheter 1327.

The occlusion device 1300 may be loaded into the catheter 1325 by axially sliding the first half 1335 relative to the second half 1337, exposing channels 1333. Thread 1331 may be looped through apex 1302 and tensioned to secure the occlusion device 1300 to push tube 1329. Thread 1331 and push tube 1329 are proximally withdrawn to load occlusion device 1300 into catheter 1325. When the occlusion device is loaded, the retention elements 1306 are contained within channels 1333. First half 1335 axially slides relative to second half 1337 to secure occlusion device 1300 in channels 1333.

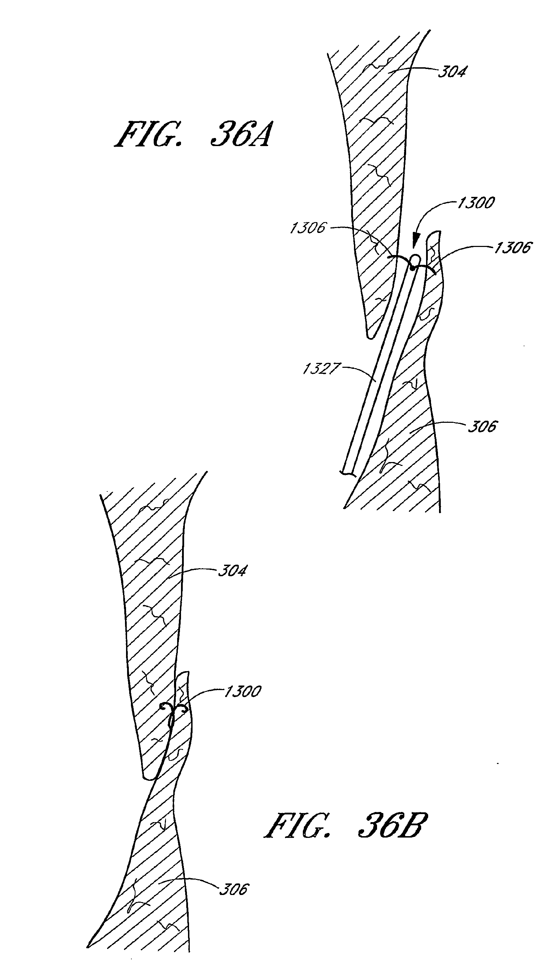

Figure 36A shows the delivery of occlusion device 1300 into septum secundum 304 and septum primum 306. Catheter 1327 is advanced into a foramen ovale such that channels 1333 are located adjacent to the septum secundum 304 and septum primum 306. Push rod 1329 is distally advanced to cause retention elements 1306 to penetrate the septa. Thread 1331 may be proximally withdrawn to disengage apex 1302 from push tube 1329. Delivery device 1325 may be proximally withdrawn to allow occlusion device 1300 to draw the septum secundum 304 and septum primum 306 together, thereby closing and sealing the patent foramen ovale 302. See Figure 36B.

In another embodiment, an occlusion device may be delivered between the septum secundum 304 and septum primum 306, preventing the septa from separating, thereby preventing a blood clot or other emboli from traversing a patent foramen ovale and entering the patients arterial circulation. An occlusion device having sharp ends and retention elements is provided. The device may be pushed axially through a catheter and delivered such that the device transversely engages the septa.

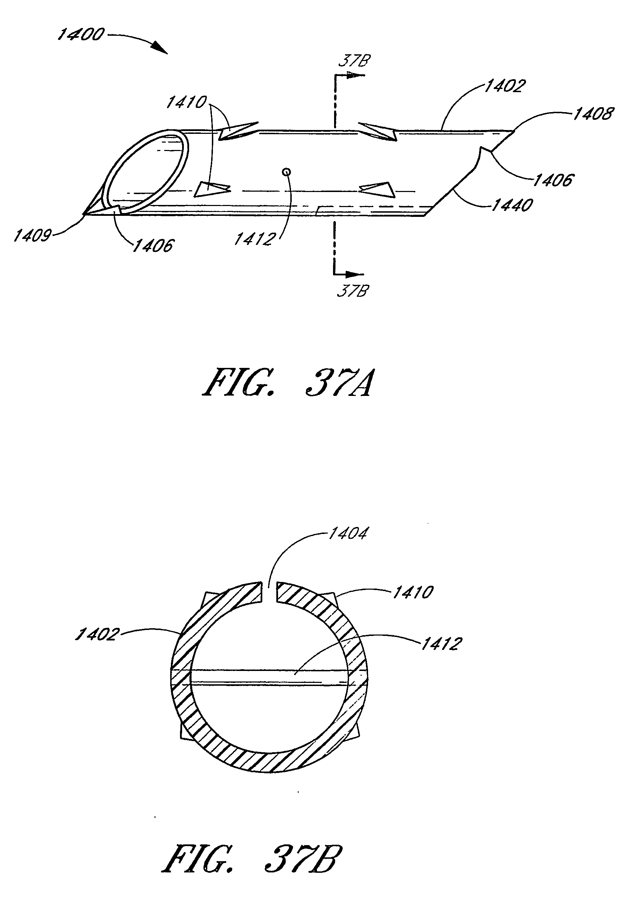

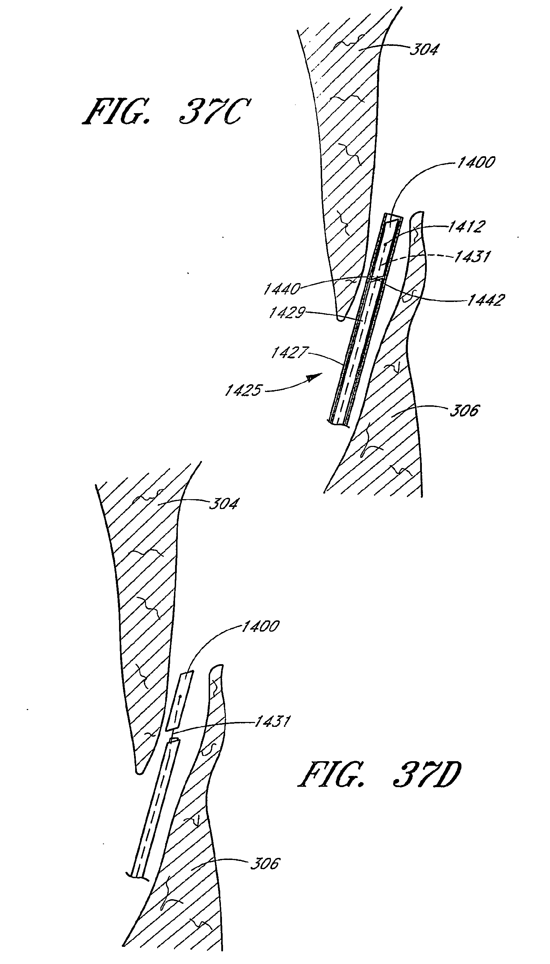

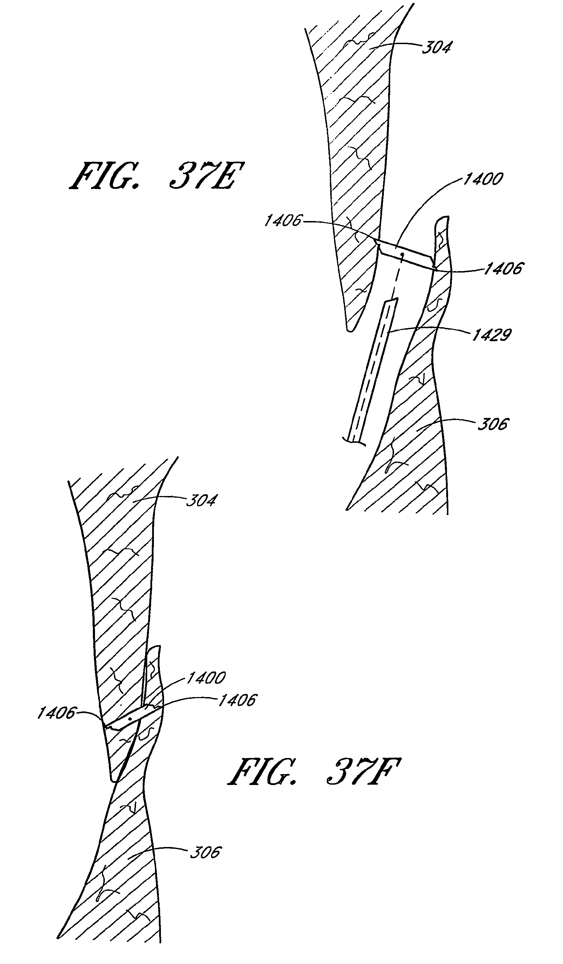

Referring to Figures 37A and 37B, an occlusion device 1400 comprises an elongate body 1402, an opening 1404, and retention elements 1406 and 1410. First end 1408 and second end 1409 may be sharpened to penetrate tissue. The elongate body may include retention elements 1406. In some embodiments, the retention element 1406 is a barb configured to resist withdrawal from a septum once the barb has engaged the septum. Occlusion device 1400 may also include retention elements 1410. Retention elements 1410 may be configured to engage the septa and resist withdrawal from a septum once the barb has engaged the septum. In some embodiments, retention elements 1410 are barbs, and may be biased to spring out from the surface of elongate body 1402 to engage the septum. Occlusion device 1400 may also include a pivot or pin 1412, as will be described hereinafter.

Occlusion device 1400 may be made from any of a variety of materials, such as a medical plastic or a metal, such as stainless steel, Nitinol, Elgiloy®, or other materials that can be determined through routine experimentation by those of skill in the art. In one embodiment, the occlusion device is laser cut from a Nitinol tube. In some embodiments, the device may be biased to self-expand upon delivery at the patent foramen ovale. In another embodiment, the occlusion device may also be biodegradable. In some embodiments, other metallic or non-metallic biocompatible materials may be used to form occlusion device 1400.

Referring to Figure 37C, a delivery system 1425 may be used to deliver occlusion device 1400 to a treatment site such as a patent foramen ovale. Delivery system 1425 includes a catheter 1427, a push tube 1429, and thread 1431. The occlusion device 1400 may be delivered through a catheter 1427. The occlusion device 1400 may be releasably attached to a push tube 1429 using any suitable mechanism, such as thread 1431 looped around a pivot 1412. Upon delivery to the patent foramen ovale, the occlusion device 1400 is pushed out of catheter 1427 to engage the septum secundum 304 and septum primum 306.

To load the occlusion device 1400 into delivery device 1425, thread 1431 may be looped around pivot 1412 of occlusion device 1400 and both ends of thread 1431 may be drawn through the interior of push tube 1429. The proximal end of push tube 1429 is loaded into the distal end of catheter 1427, and the slotted end of elongate body 1402 is advanced into the distal end of catheter 1427 while applying tension to thread 1431 to secure the occlusion device 1400 adjacent to push tube 1429. Optional retention elements 1429 at the non-slotted end of the elongate body 1402 may be radially compressed to facilitate their introduction into catheter 1427. Thread 1431 and push tube 1429 are drawn proximally together to fully load occlusion device 1400 into catheter 1427. When loaded, retention elements 1410 may be elastically compressed within the lumen of catheter 1427.

Catheter 1427 may be manufactured in accordance with any of a variety of techniques. In one embodiment, the catheter 1427 may be extruded from any of a variety of materials, such as HDPE, PEBAX®, nylon and PEEK^{™}. In some embodiments, at least a portion of or all of the length of the catheter body may comprise a spring coil, solid walled hypodermic needle or other metal tubing, or a braided reinforced wall, as are known in the art. The spring coil, tubing, braided reinforcement, or other structures may be encapsulated with thermoset polymers such as polyimide and the like or with thermoplastic polymers such as PEBAX® and the like.

The push tube 1429 desirably has good column strength. Push tube 1429 may be formed from any of a variety of ways, such as a spring coil, solid walled hypodermic needle or other metal tubing, or a braided reinforced wall, as are known in the art. The spring coil, tubing, braided reinforcement, or other structures may be encapsulated with thermoset polymers such as polyimide or thermoplastic polymers such as PEEK^{™}, and the like.

The thread 1431 may be manufactured from a variety of high strength flexible materials such as metal wire or cable, Kevlar, polyester thread, oriented ultra-high molecular weight polyethylene, and the like.

Figures 37C-F show a method of delivering occlusion device 1400 to the septum secundum 304 and septum primum 306. Delivery device 1425 is advanced to a patent foramen ovale, such that occlusion device 1400 is located adjacent the septum secundum 304 and septum primum 306. Catheter 1427 is proximally withdrawn while holding push tube 1429 and thread 1431 stationary to fully expose occlusion device 1400.

Thread 1431 passes through slot 1404 to allow full exposure of occlusion device 1400. Both ends of thread 1431 are withdrawn proximally while holding push tube 1429 stationary to cause the proximally sloped end 1440 of the elongate body 1402 to slide against the distally sloped end 1442 of the push tube 1429, thereby causing the occlusion device 1400 to pivot relative to catheter 1427 and push tube 1429. Further proximal retraction of both ends of thread 1431 causes occlusion device to continue to rotate relative to axis of catheter 1427 until retention element 1406 penetrates into the septum secundum 304. Delivery device 1425 may be partially proximally withdrawn to assist in engaging retention element 1406 with septum secundum 304. Distal retention element 1406 engages with the septum primum 306 when the distal retention element 1406 is exposed. Delivery device 1425 may be partially proximally withdrawn to assist in engaging distal retention element 1406 with the septum primum 306, using the elasticity of the septa to advancing in engaging the retention elements. Improved engagement of optional retention elements 1420 may be achieved by proximally and distally moving the delivery device 1425. Thread 1431 may be proximally withdrawn to disengage from pivot 1412 by proximally pulling one end of thread 1431 from push tube 1429. Delivery device 1425 may be proximally withdrawn from the patient, leaving occlusion device 1400 in place to prevent separation of the septum secundum 304 and septum primum 306.

Although this invention has been disclosed in the context of certain preferred embodiments and examples, it will be understood by those skilled in the art that the present invention extends beyond the specifically disclosed embodiments to other alternative embodiments and/or uses of the invention and obvious modifications and equivalents thereof. In addition, while a number of variations of the invention have been shown and described in detail, other modifications, which are within the scope of this invention, will be readily apparent to those of skill in the art based upon this disclosure. It is also contemplated that various combinations or subcombinations of the specific features and aspects of the embodiments may be made and still fall within the scope of the invention. Accordingly, it should be understood that various features and aspects of the disclosed embodiments can be combined with or substituted for one another in order to form varying modes of the disclosed invention. Thus, it is intended that the scope of the present invention herein disclosed should not be limited by the particular disclosed embodiments described above, but should be determined only by the scope of the claims.

## Claims

1. A system for closing a patent foramen ovale, comprising:
a delivery catheter (308) having a proximal end (310) and a distal end (312) and a passageway (316) extending therethrough;
a tissue piercing structure (320) having an opening (326) and coupled to a first actuator (328), the tissue piercing structure (320) being slideable within the passageway (316) of the delivery catheter (308) and being configured to pierce through tissue at the patent foramen ovale;
a closure device (200) configured to be received within the opening (326) of the tissue piercing structure (320), the closure device (200) comprising:
an elongate body (206) having a proximal end and a distal end;
an expandable distal retaining portion (208) at the distal end of the elongate body (206), the expandable distal retaining portion (208) with at least a portion thereof having a dimension that, when expanded, exceeds that of a portion of the tissue piercing structure and is configured to engage tissue on one side of the patent foramen ovale; and
a proximal retaining portion (204) having an aperture (214) extending at least partially therethrough and being configured to engage the elongate body (206) at its proximal end and being configured to engage tissue on another side of the patent foramen ovale; and
a second actuator (329) releasably engaged with the proximal end of the elongate body (206) and adapted to move at least the elongate body (206) and the distal retaining portion (208) relative to the tissue piercing structure (320);
wherein the proximal retaining portion (204) is configured to slide axially at least partially along the elongate body (206) to engage the elongate body (206).

2. The system of Claim 1, wherein the proximal retaining portion (204) is expandable when outside of the tissue piercing structure (320).

3. The system of Claims 1 or 2, wherein the proximal retaining portion (204), when in an unexpanded condition, is generally cylindrical.

4. The system of Claims 1-3, wherein the aperture (214) that extends through at least a portion of the proximal retaining portion (204) further comprises a plurality of tines (216).

5. The system of any of the preceding claims, wherein the distal retaining portion (208) is configured to extend in a direction substantially parallel to an axis of the elongate member (206) when in the opening (326) of the tissue piercing structure (320).

6. The system of any of the preceding claims, wherein the distal retaining portion (208) is configured to extend in a direction that is substantially perpendicular to an axis of the elongate member (206) when the distal retaining portion (208) is in its expanded condition.

7. The system of Claim 1, wherein the distal retaining portion (208) has a longitudinal axis that, in an expanded condition of the distal retaining portion (208), is at an angle with respect to a longitudinal axis of the elongate member (206).

8. The system of Claim 7, wherein the angle is between about 15 degrees and about 165 degrees.

9. The system of any of the preceding claims, wherein the dimension of the distal retaining portion (208) is between about 1 centimeter and about 5 centimeters when expanded.

10. The system of any of the preceding claims, wherein the first actuator (328) is spring activated to move the tissue piercing structure (320) relative to the delivery catheter (308).

11. The system of any of the preceding claims, wherein the proximal retaining portion (204) is adapted to slide over the second actuator (329).

12. The system of any of the preceding claims, further comprising a third actuator (325) adapted to move the proximal retaining portion (204) distally relative to the elongate body (206).

13. The system of any of the preceding claims, wherein the second actuator (329) extends through the third actuator (325).

## Patentansprüche

1. System zum Verschließen eines offenen Foramen ovale, wobei das System folgendes beinhaltet:
einen Zuführungskatheter (308) mit einem proximalen Ende (310) und einem distalen Ende (312) und einem sich durch diesen hindurch erstreckenden Durchgang (316),
eine Gewebe durchstechende Struktur (320), die eine Öffnung (326) aufweist und mit einer ersten Betätigungsvorrichtung (328) verbunden ist, wobei die Gewebe durchstechende Struktur (320) innerhalb des Durchgangs (316) des Zuführungskatheters (308) verschiebbar angeordnet und so ausgestaltet ist, daß sie an dem offenen Foramen ovale Gewebe durchsticht,
eine Verschließvorrichtung (200), die dafür ausgestaltet ist, in der Öffnung (326) der Gewebe durchstechenden Struktur (320) aufgenommen zu werden, wobei die Verschließvorrichtung (200) folgendes aufweist:
einen länglichen Körper (206) mit einem proximalen Ende und einem distalen Ende,
einen ausdehnbaren bzw. verlängerbaren distalen Halteabschnitt (208) am distalen Ende des länglichen Körpers (206), wobei wenigstens ein Abschnitt des ausdehnbaren distalen Halteabschnitts (208) eine Dimension hat, die im ausgedehnten Zustand die Dimension eines Abschnitts der Gewebe durchstechenden Struktur übersteigt und so ausgestaltet ist, daß sie mit Gewebe auf einer Seite des offenen Foramen ovale in Eingriff tritt, und
einen proximalen Halteabschnitt (204) mit einer Öffnung (214), die sich wenigstens teilweise durch diesen hindurch erstreckt und so ausgestaltet ist, daß sie mit dem länglichen Körper (206) an dessen proximalem Ende in Eingriff tritt, und so ausgestaltet ist, daß sie mit Gewebe auf einer anderen Seite des offenen Foramen ovale in Eingriff tritt, und
eine zweite Betätigungsvorrichtung (329), die mit dem proximalen Ende des länglichen Körpers (206) lösbar in Eingriff steht und so ausgestaltet ist, daß sie wenigstens den länglichen Körper (206) und den distalen Halteabschnitt (208) relativ zu der Gewebe durchstechenden Struktur (320) bewegt,
wobei der proximale Halteabschnitt (204) so ausgestaltet ist, daß er in axialer Richtung wenigstens teilweise entlang des länglichen Körpers (206) gleitet, um mit dem länglichen Körper (206) in Eingriff zu treten.

2. System nach Anspruch 1, wobei der proximale Halteabschnitt (204) ausdehnbar ist, wenn er sich außerhalb der Gewebe durchstechenden Struktur (320) befindet.

3. System nach einem der Ansprüche 1 oder 2, wobei der proximale Halteabschnitt (204) im nicht-ausgedehnten Zustand im allgemeinen zylindrisch ist.

4. System nach einem der Ansprüche 1 bis 3, wobei die Öffnung (214), die sich durch wenigstens einen Abschnitt des proximalen Halteabschnitts (204) erstreckt, weiterhin eine Mehrzahl von Zacken (216) aufweist.

5. System nach einem der vorangegangenen Ansprüche, wobei der distale Halteabschnitt (208) so ausgestaltet ist, daß er sich in einer Richtung im wesentlichen parallel zu einer Achse des länglichen Teils (206) erstreckt, wenn er sich in der Öffnung (326) der Gewebe durchstechenden Struktur (320) befindet.

6. System nach einem der vorangegangenen Ansprüche, wobei der distale Halteabschnitt (208) so ausgestaltet ist, daß er sich in einer Richtung im wesentlichen senkrecht zu einer Achse des länglichen Teils (206) erstreckt, wenn sich der distale Halteabschnitt (208) in seinem ausgedehnten Zustand befindet.

7. System nach Anspruch 1, wobei der distale Halteabschnitt (208) eine Längsachse hat, die in einem ausgedehnten Zustand des distalen Halteabschnitts (208) in einem Winkel in Bezug auf eine Längsachse des länglichen Teils (206) steht.

8. System nach Anspruch 7, wobei der Winkel zwischen etwa 15 Grad und etwa 165 Grad beträgt.

9. System nach einem der vorangegangenen Ansprüche, wobei die Dimension des distalen Halteabschnitts (208) zwischen etwa 1 Zentimeter und etwa 5 Zentimeter beträgt, wenn er ausgedehnt ist.

10. System nach einem der vorangegangenen Ansprüche, wobei die erste Betätigungsvorrichtung (328) federaktiviert ist, um die Gewebe durchstechende Struktur (320) relativ zu dem Zuführungskatheter (308) zu bewegen.

11. System nach einem der vorangegangenen Ansprüche, wobei der proximale Halteabschnitt (204) so ausgestaltet ist, daß er über die zweite Betätigungsvorrichtung (329) gleitet.

12. System nach einem der vorangegangenen Ansprüche, welches weiterhin eine dritte Betätigungsvorrichtung (325) aufweist, die so ausgestaltet ist, daß sie den proximalen Halteabschnitt (204) in distaler Richtung relativ zu dem länglichen Körper (206) bewegt.

13. System nach einem der vorangegangenen Ansprüche, wobei die zweite Betätigungsvorrichtung (329) sich durch die dritte Betätigungsvorrichtung (325) erstreckt.

## Revendications

1. Système de fermeture de la persistance du foramen ovale, comprenant :
un cathéter de délivrance (308) présentant une extrémité proximale (310) et une extrémité distale (312) et une voie de passage (316) s'étendant au travers de celui-ci,
une structure de percement de tissu (320) présentant une ouverture (326) et reliée à un premier actionneur (328), la structure de percement de tissu (320) pouvant coulisser à l'intérieur de la voie de passage (316) du cathéter de délivrance (308) et étant configurée pour percer à travers un tissu au niveau de la persistance du foramen ovale,
un dispositif de fermeture (200) configuré pour être reçu à l'intérieur de l'ouverture (326) de la structure de percement de tissu (320), le dispositif de fermeture (200) comprenant :
un corps allongé (206) présentant une extrémité proximale et une extrémité distale,
une partie de retenue distale extensible (208) au niveau de l'extrémité distale du corps allongé (206), la partie de retenue distale extensible (208) comportant au moins une partie de celle-ci ayant une dimension qui, lorsqu'elle est étendue, dépasse celle d'une partie de la structure de percement de tissu et est configurée pour engager le tissu d'un côté de la persistance du foramen ovale, et
une partie de retenue proximale (204) ayant une ouverture (214) s'étendant au moins partiellement au travers de celle-ci et étant configurée pour engager le corps allongé (206) au niveau de son extrémité proximale et étant configurée pour engager le tissu d'un autre côté de la persistance du foramen ovale, et
un deuxième actionneur (329) engagé de manière libérable avec l'extrémité proximale du corps allongé (206) et conçu pour déplacer au moins le corps allongé (206) et la partie de retenue distale (208) par rapport à la structure de percement de tissu (320),
où la partie de retenue proximale (204) est configurée pour coulisser axialement au moins partiellement le long du corps allongé (206) pour engager le corps allongé (206).

2. Système selon la revendication 1, dans lequel la partie de retenue proximale (204) est extensible lorsqu'elle est à l'extérieur de la structure de percement de tissu (320).

3. Système selon la revendication 1 ou 2, dans lequel la partie de retenue proximale (204), lorsqu'elle est dans un état non étendu, est globalement cylindrique.

4. Système selon les revendications 1 à 3, dans lequel l'ouverture (214), qui s'étend au travers d'au moins une partie de la partie de retenue proximale (204), comprend en outre une pluralité de dents (216).

5. Système selon l'une quelconque des revendications précédentes, dans lequel la partie de retenue distale (208) est configurée pour s'étendre dans une direction pratiquement parallèle à un axe de l'élément allongé (206) lorsque celui-ci est dans l'ouverture (326) de la structure de percement de tissu (320).

6. Système selon l'une quelconque des revendications précédentes, dans lequel la partie de retenue distale (208) est configurée pour s'étendre dans une direction qui est sensiblement perpendiculaire à un axe de l'élément allongé (206) lorsque la partie de retenue distale (208) est dans son état étendu.

7. Système selon la revendication 1, dans lequel la partie de retenue distale (208) présente un axe longitudinal qui, dans un état étendu de la partie de retenue distale (208), est à un certain angle par rapport à l'axe longitudinal de l'élément allongé (206).

8. Système selon la revendication 7, dans lequel l'angle est compris entre environ 15 degrés et environ 165 degrés.

9. Système selon l'une quelconque des revendications précédentes, dans lequel la dimension de la partie de retenue distale (208) est comprise entre environ 1 centimètre et environ 5 centimètres lorsqu'elle est étendue.

10. Système selon l'une quelconque des revendications précédentes, dans lequel le premier actionneur (328) est activé par ressort pour déplacer la structure de percement de tissu (320) par rapport au cathéter de délivrance (308).

11. Système selon l'une quelconque des revendications précédentes, dans lequel la partie de retenue proximale (204) est adaptée pour coulisser sur le deuxième actionneur (329).

12. Système selon l'une quelconque des revendications précédentes, comprenant en outre un troisième actionneur (325) conçu pour déplacer la partie de retenue proximale (204) de manière distale par rapport au corps allongé (206).

13. Système selon l'une quelconque des revendications précédentes, dans lequel le deuxième actionneur (329) s'étend au travers du troisième actionneur (325).
